# EUROPEAN PATENT SPECIFICATION

(11) **EP 1 904 057 B1**
(45) Date of publication and mention of the grant of the patent: **16.12.2009**
(21) Application number: 06787067.5
(22) Date of filing: 13.07.2006
(51) Int. Cl.: A61K 31/395, A61P 35/00, A61P 11/06, A61P 37/08

(54) **HYDROQUINONE ANSAMYCINS IN THE TREATMENT OF GASTROINTESTINAL STROMAL TUMORS**
HYDROCHINON- ANSAMYCINEN IN DER BEHANDLUNG VON GASTROINTESTINALEN STROMATUMOREN
ANSAMYCINES D'HYDROQUINONE POUR LE TRAITEMENT DE TUMEURS DU STROMA GASTRO-INTESTINAL

(30) Priority: 13.07.2005 US 180314
(43) Date of publication of application: 02.04.2008
(73) Proprietor: Infinity Discovery, Inc., Cambridge, MA 02139 (US)
(72) Inventor: ADAMS, Julian, Boston, MA 02116 (US); BRAIN, Julia, Charlestown, MA 02129 (US); GAO, Yun, Southborough, MA 01772 (US); GEORGES-EVANGELINOS, Asimina, T., Andover, MA 01810 (US); GRAYZEL, David, Winchester, MA 01890 (US); GRENIER, Louis, Brookline, MA 02445 (US); NORMANT, Emmanuel, Winchester, MA 01890 (US); PAK, Roger, H., Boxborough, MA 01719 (US); PALOMBELLA, Vito, Needham, MA 02492 (US); PORTER, James, R., Brighton, MA 02135 (US); TONG, Jeffrey, K., Boston, MA 02116 (US); WRIGHT, James, L., Lexington, MA 02421 (US)
(74) Representative: Atkinson, Jonathan David Mark
(86) International application number: PCT/US2006/027113
(87) International publication number: WO 2007/009007

(56) References cited:
- WO-A-93/14215
- WO-A-95/01342
- WO-A-03/013430
- WO-A-2005/063714
- GB-A- 2 106 111

## Description

### BACKGROUND OF THE INVENTION

Heat shock protein 90 ("Hsp90") is a highly abundant protein which is essential for cell viability and exhibits dual chaperone functions *(*J. Cell Biol. (2001) 154:267-273, Trends Biochem. Sci. (1999) 24:136-141). It plays a key role in the cellular stress response by interacting with many proteins after their native conformation has been altered by various environmental stresses, such as heat shock, ensuring adequate protein folding and preventing non-specific aggregation *(*Pharmacological Rev. (1998) 50:493-513). In addition, recent results suggest that Hsp90 may also play a role in buffering against the effects of mutation, presumably by correcting the inappropriate folding of mutant proteins *(*Nature (1998) 396:336-342).-However, Hsp90 also has an important regulatory role under normal physiological conditions and is responsible for the conformational stability and maturation of a number of specific client proteins, of which about 40 are known (S*ee*, Expert. Opin. Biol Ther. (2002) 2: 3-24). Hsp90 antagonists are currently being explored in a large number of biological contexts where a therapeutic effect can be obtained for a condition or disorder by inhibiting one or more aspects of Hsp90 activity. Although the primary focus has been on proliferative disorders, such as cancers, the use of Hsp90 antagonists to treat other conditions is being explored.

Geldanamycin is a macrocyclic lactam that is a member of the benzoquinone-containing ansamycins family of natural products. Geldanamycin's nanomolar potency and apparent selectivity for killing tumor cells, as well as the discovery that its primary target in mammalian cells is Hsp90, has stimulated interest in its development as an anti-cancer drug. However, the extremely low solubility of these molecules and the association of hepatotoxicity with the administration of geldanamycin has led to difficulties in developing an approvable agent for therapeutic applications. In particular, geldanamycin has poor water solubility, making it difficult to deliver in therapeutically effective doses. Consequently, there remains a need to discover more soluble analogs of benzoquinone-containing ansamycins.

### SUMMARY OF THE INVENTION

The present invention is relates to methods for treating and modulating disorders associated with hyperproliferation, such as cancer.

According to one aspect of the invention there is provided the use of a hydroquinone ansamycin compound represented by formula **1**: 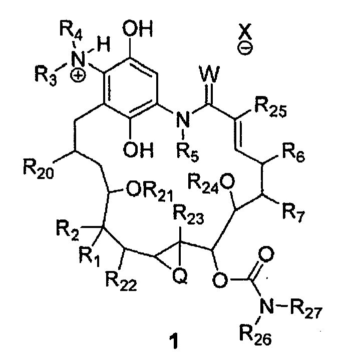 wherein independently for each occurrence: W is oxygen or sulfur; Q is oxygen, NR, N(acyl) or a bond; X⁻ is a conjugate base of a Pharmaceutical acceptable acid; R for each occurrence is independently selected from the group consisting of hydrogen, alkyl, cycloalkyl, heterocycloalkyl, aralkyl, heteroaryl, and heteroaralkyl; R₁ is hydroxyl, alkoxyl, -OC(O)R₈, -OC(O)OR₉, -OC(O)NR₁₀R₁₁, -OSO₂R₁₂, -OC(O)NHSO₂NR₁₃R₁₄**,** -NR₁₃R₁₄, or halide; and R₂ is hydrogen, alkyl, or aralkyl; or R₁ and R₂ taken together, along with the carbon to which they are bonded, represent -(C=O)-, -(C=N-OR)-, -(C=N-NHR)-, or - (C=N-R)-; R₃ and R₄ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ)-R₁₆; or R₃ taken together with R₄ represent a 4-8 membered optionally substituted heterocyclic ring; R₅ is selected from the group consisting of H, alkyl, aralkyl, and a group having the formula **1a**: 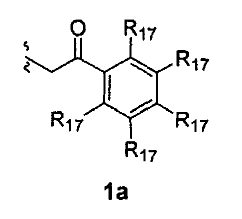 wherein R₁₇ is selected independently from the group consisting of hydrogen, halide, hydroxyl, alkoxyl, aryloxy, acyloxy, amino, alkylamino, arylamino, acylamino, aralkylamino, nitro, acylthio, carboxamide, carboxyl, nitrile, -COR₁₈, -CO₂R₁₈, -N(R₁₈)CO₂R₁₉, -OC(O)N(R₁₈)(R₁₉), -N(R₁₈)SO₂R₁₉, -N(R₁₈)C(O)N(R₁₈)(R₁₉), and -CH₂O-heterocyclyl; R₆ and R₇ are both hydrogen; or R₆ and R₇ taken together form a bond; R₈ is hydrogen, alkyl, alkenyl, alkynyl, aryl, cycloalkyl, heterocycloalkyl, aralkyl, heteroaryl, heteroaralkyl, or -[(CR₂)ₚ]-R₁₆; R₉ is alkyl, alkenyl, alkynyl, aryl, cycloalkyl, heterocycloalkyl, aralkyl, heteroaryl, heteroaralkyl, or -[(CR₂)ₚ]-R₁₆; R₁₀ and R₁₁ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ]-R₁₆; or R₁₀ and R₁₁ taken together with the nitrogen to which they are bonded represent a 4-8 membered optionally substituted heterocyclic ring; R₁₂ is alkyl, alkenyl, alkynyl, aryl, cycloalkyl, heterocycloalkyl, aralkyl, heteroaryl, heteroaralkyl, or -((CR₂)ₚ]-R₁₆; R₁₃ and R₁₄ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ]-R₁₆; or R₁₃ and R₁₄ taken together with the nitrogen to which they are bonded represent a 4-8 membered optionally substituted heterocyclic ring; R₁₆ for each occurrence is independently selected from the group consisting of hydrogen, hydroxyl, acylamino, -N(R₁₈)COR₁₉, -N(R₁₈)C(O)OR₁₉, -N(R₁₈)SO₂(R₁₉), -CON(R₁₈)(R₁₉), - OC(O)N(R₁₈)(R₁₉), -SO₂N(R₁₈)(R₁₉), -N(R₁₈)(R₁₉), -OC(O)OR₁₈, -COOR₁₈, -C(O)N(OH)(R₁₈), -OS(O)₂OR₁₈, -S(O)₂OR₁₈, -OP(O)(OR₁₈)(OR₁₉), -N(R₁₈)P(O)(OR₁₈)(OR₁₉), and -P(O)(OR₁₈)(OR₁₉); p is 1, 2, 3, 4, 5, or 6; R₁₈ for each occurrence is independently selected from the group consisting of hydrogen, alkyl, aryl, cycloalkyl, heterocycloalkyl, aralkyl, heteroaryl, and heteroaralkyl; R₁₉ for each occurrence is independently selected from the group consisting of hydrogen, alkyl, aryl, cycloalkyl, heterocycloalkyl, aralkyl, heteroaryl, and heteroaralkyl; or R₁₈ taken together with R₁₉ represent a 4-8 membered optionally substituted ring; R₂₀, R₂₁, R₂₂, R_{24,} and R₂₅, for each occurrence are independently alkyl; R₂₃ is alkyl, -CH₂OH, -CHO, -COOR₁₈, or - CH(OR₁₈)₂; R₂₆ and R₂₇ for each occurrence are independently selected from the group consisting of hydrogen, alkyl, aryl, cycloalkyl, heterocycloalkyl, aralkyl, heteroaryl, and heteroaralkyl; and the absolute stereochemistry at a stereogenic center of formula **1** may be R or S or a mixture thereof and the stereochemistry of a double bond may be E or Z or a mixture thereof; in the manufacture of a medicament for use in the treatment of one or more gastrointestinal stromal tumors in a mammal.

In one aspect, the invention features a method of treating an abnormal condition in a mammal, where the abnormal condition is associated with an aberration in a signal transduction pathway mediated by a cKit kinase. The method comprises the step of administering to the mammal a therapeutically effective amount of a hydroquinone ansamycin compound. The abnormal condition is the presence of one or more gastrointestinal stromal tumors.

In another aspect the invention features a method of treating an abnormal condition in a mammal, where the abnormal condition is associated with an aberration in a signal transduction pathway mediated by PDGFRα, where the method comprises the step of administering to the mammal a therapeutically effective amount of a hydroquinone ansamycin compound.

In another aspect, the invention features a method of treating a hyperproliferative disorder, comprising the step of administering to a mammal a therapeutically effective amount of a hydroquinone ansamycin compound, where the hyperproliferative disorder is associated with an aberration in a signal transduction pathway mediated by a mutant protein; or by a mutant gain-of-function protein, eg EGFR, VEGFR, Flt-3, Her-2, or b-Raf. The disorder is the presence of at least one gastrointestinal stromal tumor.

The aspects of the invention can include one or more of the following features: the mammal can be a human; the mode of administration can be by inhalation, oral, intravenous, sublingual, ocular, transdermal, rectal, vaginal, topical, intramuscular, intraarterial, intrathecal, subcutaneous, buccal administration.

In particular, the hydroquinone ansamycin compound can be 17-allylamino-17-demethoxy-18,21-dihydrogeldanamycin, or a pharmaceutically acceptable salt thereof, such as the hydrochloride salt of 17-allylamino-17-demethoxy-18,21-dihydrogeldanamycin.

### DETAILED DESCRIPTION OF THE INVENTION

### Definitions

The definitions of terms used herein are meant to incorporate the present state-of-the-art definitions recognised for each term in the chemical and pharmaceutical fields. Where appropriate, exemplification is provided. The definitions apply to the terms as they are used throughout this specification, unless otherwise limited in specific instances, either individually or as part of a larger group.

Where stereochemistry is not specifically indicated, all stereoisomers of the inventive compounds are included within the scope of the invention, as pure compounds as well as mixtures thereof. Unless otherwise indicated, individual enantiomers, diastereomers, geometrical isomers, and combinations and mixtures thereof are all encompassed by the present invention. Polymorphic crystalline forms and solvates are also encompassed within the scope of this invention.

As used herein, the term "benzoquinone ansamycin" refers to a compound comprising a macrocyclic lactam, further comprising only one lactam in the ring and a benzoquinone moiety in the lactam ring, wherein the benzoquinone moiety has at least one nitrogen substituent, wherein one of the at least one nitrogen substitutents is part of said only one amide moiety in the lactam ring. Specific examples of naturally-occurring benzoquinone ansamycins include, but are not limited to, geldanamycin and herbimycin. The term "geldanamycin analog" refers to a benzoquinone ansamycin that can be derived from geldanamycin e.g., by chemical manipulation; for example 17-allylamino-17-demethoxygeldanamycin (17-AAG) or 17-(2-dimethylaminoethy)amino-17-demethoxygeldanamycin (17-DMAG). As used herein, the term "hydroquinone ansamycin" refers to a benzoquinone ansamycin in which the benzoquinone moiety has been reduced (a two-electron reduction) to the corresponding hydroquinone moiety. An example of such a reduction in a simplified system is the two-electron reduction of benzoquinone to hydroquinone.

As used herein, the term "isolated" means the compound is not in a cell or organism and the compound is separated from some or all of the components that typically accompany it in nature.

As used herein, the term "pure" means the isolated sample contains at least 60% by weight of the compound. Preferably, the isolated sample contains at least 70% by weight of the compound. More preferably, the isolated sample contains at least 80% by weight of the compound. Even more preferably, the isolated sample contains at least 90% by weight of the compound. Most preferably, the isolated sample contains at least 95% by weight of the compound. The purity of an isolated sample of a compound may be assessed by a number of methods or a combination of them; e.g., thin-layer, preparative or flash chromatography, mass spectrometry, HPLC, NMR analysis, and the like.

The term "heteroatom" is art-recognized and refers to an atom of any element other than carbon or hydrogen. Illustrative heteroatoms include boron, nitrogen, oxygen, phosphorus, sulfur and selenium.

The term "alkyl" is art-recognized, and includes saturated aliphatic groups, including straight-chain alkyl groups, branched-chain alkyl groups, cycloalkyl (alicyclic), groups, alkyl substituted cycloalkyl groups, and cycloalkyl substituted alkyl groups. In certain embodiments, a straight chain or branched chain alkyl has about 30 or fewer carbon atoms in its backbone (e.g., C₁-C₃₀ for straight chain, C₃-C₃₀ for branched chain), and alternatively, about 20 or fewer. Likewise, cycloalkyls have from about 3 to about 10 carbon atoms in their ring structure, and alternatively about 5, 6 or 7 carbons in the ring structure.

Unless the number of carbons is otherwise specified, "lower alkyl," refers to an alkyl group, as defined above, but having from one to about ten carbons, alternatively from one to about six carbon atoms in its backbone structure. Likewise, "lower alkenyl," and "lower alkynyl" have similar chain lengths.

The term "aralkyl" is art-recognized and refers to an alkyl group substituted with an aryl group (e.g., an aromatic or heteroaromatic group).

The terms "alkenyl" and "alkynyl" are art-recognized and refer to unsaturated aliphatic groups analogous in length and possible substitution to the alkyls described above, but that contain at least one double or triple bond respectively.

The term "aryl" is art-recognized and refers to 5-, 6- and 7-membered single-ring aromatic groups that may include from zero to four heteroatoms, for example, benzene, naphthalene, anthracene, pyrene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "aryl heterocycles" or "heteroaromatics." The aromatic ring may be substituted at one or more ring positions with such substituents as described above, for example, halogen, azide, alkyl, aralkyl, alkenyl, alkenyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamide, ketone, aldehyde, ester, heterocyclyl, aromatic or heteroaromatic moieties, - CF₃, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings may be cycloalkyls, cycloalkenyls, cycloalkynyls, aryls and/or heterocyclyls.

The terms "heterocyclyl", "heteroaryl", or "heterocyclic group" are art-recognized and refer to 3- to about 10-membered ring structures, alternatively 3- to about 7-membered rings, whose ring structures include one to four heteroatoms. Heterocycles may also be polycycles. Heterocyclyl groups include, for example, thiophene, thianthrene, furan, pyran, isobenzofuran, chromene, xanthene, phenoxanthene, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring may be substituted at one or more positions with such substituents as described above, as for example, halogen, alkyl, aralkyl, alkenyl, alkynyl, cycloalkyl, hydroxyl, amino; nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The term "mutated," generally means that the activity of a protein is lower or higher than the wild-type protein. "Mutated" can also mean that a protein is changed such that it can no longer interact with a natural binding partner.

The term "optionally substituted" refers to a chemical group, such as alkyl, cycloalkyl aryl, and the like, wherein one or more hydrogen may be replaced with a with a substituent as described herein, for example, halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, -CF₃, -CN, or the like

The terms "polycyclyl" or "polycyclic group" are art-recognized and refer to two or more rings (e.g., cycloalkyls, cycloalkenyls, cycloalkynyls, aryls and/or heterocyclyls) in which two or more carbons are common to two adjoining rings, e.g., the rings are "fused rings". Rings that are joined through non-adjacent atoms are termed "bridged" rings. Each of the rings of the polycycle may be substituted with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The term "carbocycle" is art-recognized and refers to an aromatic or non-aromatic ring in which each atom of the ring is carbon.

The term "nitro" is art-recognized and refers to -NO2; the term "halogen" is art-recognized and refers to -F, -Cl, -Br or -I; the term "sulfhydryl" is art-recognized and refers to -SH; the term "hydroxyl" means -OH; and the term "sulfonyl" is art-recognized and refers to -SO₂⁻. "Halide" designates the corresponding anion of the halogens, and "pseudohalide" has the definition set forth on 560 of "Advanced Inorganic Chemistry" by Cotton and Wilkinson.

The terms "amine" and "amino" are art-recognized and refer to both unsubstituted and substituted amines, e.g., a moiety that may be represented by the general formulas: 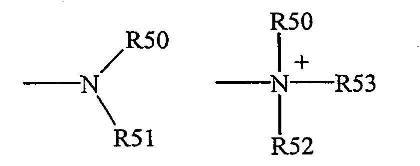 wherein R50, R51 and R52 each independently represent a hydrogen, an alkyl, an alkenyl, -(CH₂)ₘ-R61, or R50 and R51, taken together with the N atom to which they are attached complete a heterocycle having from 4 to 8 atoms in the ring structure; R61 represents an aryl, a cycloalkyl, a cycloalkenyl, a heterocycle or a polycycle; and m is zero or an integer in the range of 1 to 8. In other embodiments, R50 and R51 (and optionally R52) each independently represent a hydrogen, an alkyl, an alkenyl, or -(CH₂)ₘ-R61. Thus, the term "alkylamine" includes an amine group, as defined above, having a substituted or unsubstituted alkyl attached thereto, i.e., at least one of R50 and R51 is an alkyl group.

The term "acylamino" is art-recognized and refers to a moiety that may be represented by the general formula: 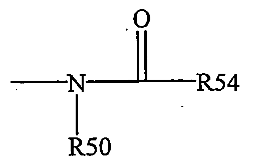 wherein R50 is as defined above, and R54 represents a hydrogen, an alkyl, an alkenyl or -(CH₂)ₘ-R61, where m and R61 are as defined above.

The term "amido" is art recognized as an amino-substituted carbonyl and includes a moiety that may be represented by the general formula: 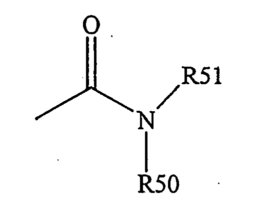 wherein R50 and R51 are as defined above. Certain embodiments of the amide in the present invention will not include imides which may be unstable.

The term "alkylthio" refers to an alkyl group, as defined above, having a sulfur radical attached thereto. In certain embodiments, the "alkylthio" moiety is represented by one of -S-alkyl, -S-alkenyl, -S-alkynyl, and -S-(CH₂)ₘ-R61, wherein m and R61 are defined above. Representative alkylthio groups include methylthio, ethyl thio, and the like.

The term "carboxyl" is art recognized and includes such moieties as may be represented by the general formulas: 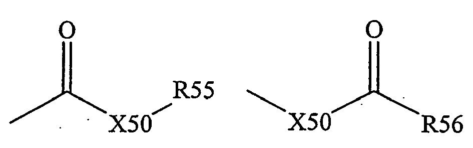 wherein X50 is a bond or represents an oxygen or a sulfur, and R55 and R56 represents a hydrogen, an alkyl, an alkenyl, -(CH₂)ₘ-R61or a pharmaceutically acceptable salt, R56 represents a hydrogen, an alkyl, an alkenyl or -(CH₂)ₘ-R61, where m and R61 are defined above. Where X50 is an oxygen and R55 or R56 is not hydrogen, the formula represents an "ester". Where X50 is an oxygen, and R55 is as defined above, the moiety is referred to herein as a carboxyl group, and particularly when R55 is a hydrogen, the formula represents a "carboxylic acid". Where X50 is an oxygen, and R56 is hydrogen, the formula represents a "formate". In general, where the oxygen atom of the above formula is replaced by sulfur, the formula represents a "thiolcarbonyl" group. Where X50 is a sulfur and R55 or R56 is not hydrogen, the formula represents a "thiolester." Where X50 is a sulfur and R55 is hydrogen, the formula represents a "thiolcarboxylic acid." Where X50 is a sulfur and R56 is hydrogen, the formula represents a "thiolformate." On the other hand, where X50 is a bond, and R55 is not hydrogen, the above formula represents a "ketone" group. Where X50 is a bond, and R55 is hydrogen, the above formula represents an "aldehyde" group.

The term "carbamoyl" refers to -O(C=O)NRR', where R and R' are independently H, aliphatic groups, aryl groups or heteroaryl groups.

The term "oxo" refers to a carbonyl oxygen (=O).

The terms "alkoxyl" or "alkoxy" are art-recognized and refer to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as may be represented by one of -O-allcyl, -O-alkenyl, -O-alkynyl, -O--(CH₂)ₘ-R61, where m and R61 are described above.

The term "sulfonate" is art recognized and refers to a moiety that may be represented by the general formula: 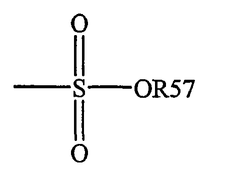 in which R57 is an electron pair, hydrogen, alkyl, cycloalkyl, or aryl.

The term "sulfate" is art recognized and includes a moiety that may be represented by the general formula: 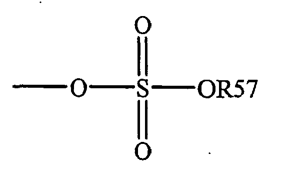 in which R57 is as defined above.

The term "sulfonamido" is art recognized and includes a moiety that may be represented by the general formula: 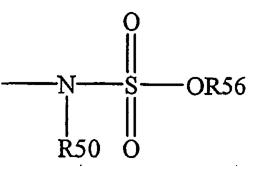 in which R50 and R56 are as defined above.

The term "sulfamoyl" is art-recognized and refers to a moiety that may be represented by the general formula: 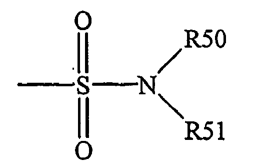 in which R50 and R51 are as defined above.

The term "sulfonyl" is art-recognized and refers to a moiety that may be represented by the general formula: 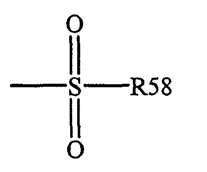 in which R58 is one of the following: hydrogen, alkyl; alkenyl, alkynyl, cycloalkyl, heterocyclyl, aryl or heteroaryl.

The term "sulfoxido" is art-recognized and refers to a moiety that may be represented by the general formula: 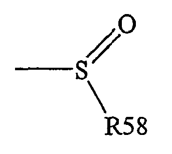 in which R58 is defined above.

Analogous substitutions may be made to alkenyl and alkynyl groups to produce, for example, aminoalkenyls, aminoalkynyls, amidoalkenyls, amidoalkynyls, iminoalkenyls, iminoalkynyls, thioalkenyls, thioalkynyls, carbonyl-substituted alkenyls or alkynyls.

The definition of each expression, e.g. alkyl, m, n, and the like, when it occurs more than once in any structure, is intended to be independent of its definition elsewhere in the same structure.

Certain compounds disclosed herein may exist in particular geometric or stereoisomeric forms, which may be optically active. The present invention contemplates all such compounds, including cis- and trans-isomers, R- and S-enantiomers, diastereomers, (D)-isomers, (L)-isomers, the racemic mixtures thereof, and other mixtures thereof. Additional asymmetric carbon atoms may be present in a substituent such as an alkyl group. All such isomers, as well as mixtures thereof, are intended to be included in this invention.

If, for instance, a particular enantiomer of a compound is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents may be one or more and the same or different for appropriate organic compounds. For purposes of this invention, the heteroatoms such as nitrogen may have hydrogen substituents and/or any permissible substituents of organic compounds described herein which satisfy the valences of the heteroatoms.

The phrase "protecting group" as used herein means temporary substituents which protect a potentially reactive functional group from undesired chemical transformations. Examples of such protecting groups include esters of carboxylic acids, silyl ethers of alcohols, and acetals and ketals of aldehydes and ketones, respectively. The field of protecting group chemistry has been reviewed (Greene, T.W.; Wuts, P.G.M. Protective Groups in Organic Synthesis, 2nd ed.; Wiley: New York, 1991).

For purposes of this invention, the chemical elements are identified in accordance with the Periodic Table of the Elements, CAS version, Handbook of Chemistry and Physics, 67th Ed., 1986-87, inside cover.

The term "pharmaceutically acceptable salt" or "salt" refers to a salt of one or more compounds. Suitable pharmaceutically acceptable salts of compounds include acid addition salts which may, for example, be formed by mixing a solution of the compound with a solution of a pharmaceutically acceptable acid, such as hydrochloric acid, hydrobromic acid, sulfuric acid, fumaric acid, maleic acid, succinic acid, benzoic acid, acetic acid, citric acid, tartaric acid, phosphoric acid, carbonic acid, or the like. Where the compounds carry one or more acidic moieties, pharmaceutically acceptable salts may be formed by treatment of a solution of the compound with a solution of a pharmaceutically acceptable base, such as lithium hydroxide, sodium hydroxide, potassium hydroxide, tetraalkylammonium hydroxide, lithium carbonate, sodium carbonate, potassium carbonate, ammonia, alkylamines, or the like.

The term "pharmaceutically acceptable acid" refers to inorganic or organic acids that exhibit no substantial toxicity. Examples of pharmaceutically acceptable acids include, but are not limited to hydrochloric acid, hydrobromic acid, sulfuric acid, nitric acid, phenylsulfonic acid, methanesulfonic acid, fumaric acid, maleic acid, succinic acid, benzoic acid, acetic acid, citric acid, tartaric acid, phosphoric acid, carbonic acid, and the like.

The term "co-salt" or "co-crystal" refers to compositions in which the reduced salt form of the ansamycin is present with at least one other salt, such as a salt of an amino acid.

The term "subject" as used herein, refers to an animal, typically a mammal or a human, that will be or has been the object of treatment, observation, and/or experiment. When the term is used in conjunction with administration of a compound or drug, then the subject has been the object of treatment, observation, and/or administration of the compound or drug.

The terms "co-administration" and "co-administering" refer to both concurrent administration (administration of two or more therapeutic agents at the same time) and time varied administration (administration of one or more therapeutic agents at a time different from that of the administration of an additional therapeutic agent or agents), as long as the therapeutic agents are present in the patient to some extent at the same time.

The term "therapeutically effective amount" as used herein, means that amount of active compound or pharmaceutical agent that elicits a biological or medicinal response in a cell culture, tissue system, animal, or human that is being sought by a researcher, veterinarian, clinician, or physician, which includes alleviation of the symptoms of the disease, condition, or disorder being treated.

The term "composition" is intended to encompass a product comprising the specified ingredients in the specified amounts, as well as any product that results, directly or indirectly, from combinations of the specified ingredients in the specified amounts, particularly co-salts such as the reduced ansamycin salt (e.g., sulfate) with a salt of an amino acid (e.g., glycine).

The term "Hsp90 mediated disorder" or "disorder mediated by cells expressing Hsp90" refers to pathological and disease conditions in which Hsp90 plays a role. Such roles can be directly related to the pathological condition or can be indirectly related to the condition. The common feature to this class of conditions is that the condition can be ameliorated by inhibiting the activity, function, or association with other proteins of Hsp90.

The term"cKit kinase" refers to a membrane receptor protein tyrosine kinase which is preferably activated upon binding Stem Cell Factor (SCF) to its extracellular domain (Yarden etal., 1987 ; Qiu etal., 1988). The receptor tyrosine kinase cKit kinase contains 5 immunoglobulin-like motifs in the extracellular domain and a cytoplasmic "split" kinase domain. The full length amino acid sequence of a cKit kinase preferably is as set forth in Yarden, et al., 1987, EMBO J. 11: 3341-3351 ; and Qiu, et al., 1988, EMBO J7: 1003-1011, which are incorporated by reference herein in their entirety, including any drawings. Mutant versions of cKit kinase are encompassed by the term"cKit kinase" and include those that fall into two classes: (1) having a single amino acid substitution, for example, at codon 816 of the human cKit kinase, or its equivalent position in other species (Ma et al., 1999, J. Invest Dermatol 112 : 165-170), and (2) those which have mutations involving the putative juxtamembrane z-helix of the protein (Ma, et al., 1999, J Biol Chem 274 : 13399-13402). Both of these publications are incorporated by reference herein in their entirety, including any drawings.

The term "pharmaceutically acceptable carrier" refers to a medium that is used to prepare a desired dosage form of a compound. A pharmaceutically acceptable carrier can include one or more solvents, diluents, or other liquid vehicles; dispersion or suspension aids; surface active agents; isotonic agents; thickening or emulsifying agents; preservatives; solid binders; lubricants; and the like. Remington's Pharmaceutical Sciences, Fifteenth Edition, E. W. Martin (Mack Publishing Co., Easton, Pa., 1975) and Handbook of Pharmaceutical Excipients, Third Edition, A. H. Kibbe ed. (American Pharmaceutical Assoc. 2000), disclose various carriers used in formulating pharmaceutical compositions and known techniques for the preparation thereof.

### Compounds

Examples of hydroquinone ansamycins include a compound of formula **1**: 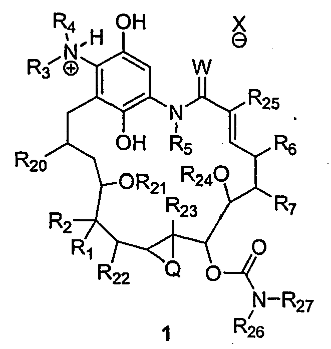 or the free base thereof;
wherein independently for each occurrence:
W is oxygen or sulfur;
Q is oxygen, NR, N(acyl) or a bond;
X⁻ is a conjugate base of a pharmaceutically acceptable acid;
R for each occurrence is independently selected from the group consisting of hydrogen, alkyl, cycloalkyl, heterocycloalkyl, aralkyl, heteroaryl, and heteroaralkyl;
R₁ is hydroxyl, alkoxyl, -OC(O)R₈, -OC(O)OR₉, -OC(O)NR₁₀R₁₁, -OSO₂R₁₂, -OC(O)NHSO₂NR₁₃R₁₄, -NR₁₃R₁₄, or halide; and R₂ is hydrogen, alkyl, or aralkyl; or R₁ and R₂ taken together, along with the carbon to which they are bonded, represent -(C=O)-, - (C=N-OR)-, -(C=N-NHR)-, or -(C=N-R)-;
R₃ and R₄ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ]-R₁₆; or R₃ taken together with R₄ represent a 4-8 membered optionally substituted heterocyclic ring;
R₅ is selected from the group consisting of H, alkyl, aralkyl, and a group having the formula **1a**: 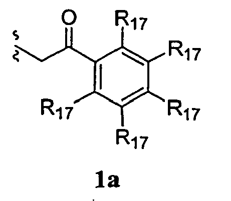
   wherein R₁₇ is selected independently from the group consisting of hydrogen, halide, hydroxyl, alkoxyl, aryloxy, acyloxy, amino, alkylamino, arylamino, acylamino, aralkylamino, nitro, acylthio, carboxamide, carboxyl, nitrile, -COR₁₈, -CO₂R₁₈, -N(R₁₈)CO₂R₁₉, -OC(O)N(R₁₈)(R₁₉), -N(R₁₈)SO₂R₁₉, -N(R₁₈)C(O)N(R₁₈)(R₁₉), and -CH₂O-heterocyclyl;
   R₆ and R₇ are both hydrogen; or R₆ and R₇ taken together form a bond;
   R₈ is hydrogen, alkyl, alkenyl, alkynyl, aryl, cycloalkyl, heterocycloalkyl, aralkyl, heteroaryl, heteroaralkyl, or -[(CR₂)ₚ]-R₁₆;
   R₉ is alkyl, alkenyl, alkynyl, aryl, cycloalkyl, heterocycloalkyl, aralkyl, heteroaryl, heteroaralkyl, or -[(CR₂)ₚ]-R₁₆;
   R₁₀ and R₁₁ are each independently selected from the group consisting of hydrogen, alkyl, alkenyl, alkynyl, aryl, cycloalkyl, heterocyloalkyl, aralkyl, heteroaryl, heteroaralkyl, and -[(CR₂)ₚ]-R₁₆; or R₁₀ and R₁₁ taken together with the nitrogen to which they are bonded represent a 4-8 membered optionally substituted heterocyclic ring;
   R₁₂ is alkyl, alkenyl, alkynyl, aryl, cycloalkyl, heterocycloalkyl, aralkyl, heteroaryl, heteroaralkyl, or -[(CR₂)ₚ]-R₁₆;
   R₁₃ and R₁₄ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ]-R₁₆; or R₁₃ and R₁₄ taken together with the nitrogen to which they are bonded represent a 4-8 membered optionally substituted heterocyclic ring;
   R₁₆ for each occurrence is independently selected from the group consisting of hydrogen, hydroxyl, acylamino, -N(R₁₈)COR₁₉, N(R₁₈)C(O)OR₁₉, -N(R₁₈)SO₂(R₁₉), -CON(R₁₈)(R₁₉), -OC(O)N(R₁₈)(R₁₉), -SO₂N(R₁₈)(R₁₉), -N(R₁₈)(R₁₉), -OC(O)OR₁₈, -COOR₁₈, -C(O)N(OH)(R₁₈), -OS(O)₂OR₁₈, -S(O)₂OR₁₈, -OP(O)(OR₁₈)(OR₁₉), -N(R₁₈)P(O)(OR₁₈)(OR₁₉), and -P(O)(OR₁₈)(OR₁₉);
   p is 1,2, 3,4, 5, or 6;
   R₁₈ for each occurrence is independently selected from the group consisting of hydrogen, alkyl, aryl, cycloalkyl, heterocycloalkyl, aralkyl, heteroaryl, and heteroaralkyl;
   R₁₉ for each occurrence is independently selected from the group consisting of hydrogen, alkyl, aryl, cycloalkyl, heterocycloalkyl, aralkyl, heteroaryl, and heteroaralkyl; or R₁₈ taken together with R₁₉ represent a 4-8 membered optionally substituted ring;
   R₂₀, R₂₁, R₂₂, R₂₄, and R₂₅, for each occurrence are independently alkyl;
   R₂₃ is alkyl, -CH₂OH, -CHO, -COOR₁₈, or -CH(OR₁₈)₂;
   R₂₆ and R₂₇ for each occurrence are independently selected from the group consisting of hydrogen, alkyl, aryl, cycloalkyl, heterocycloalkyl, aralkyl, heteroaryl, and heteroaralkyl;
   provided that when R₁ is hydroxyl, R₂ is hydrogen, R₆ and R₇ taken together form a double bond, R₂₀ is methyl, R₂₁ is methyl, R₂₂ is methyl, R₂₃ is methyl, R₂₄ is methyl, R₂₅ is methyl, R₂₆ is hydrogen, R₂₇ is hydrogen, Q is a bond, and W is oxygen; R₃ and R₄ are not both hydrogen nor when taken together represent an unsubstituted azetidine; and
   the absolute stereochemistry at a stereogenic center of formula 1 may be R or S or a mixture thereof and the stereochemistry of a double bond may be E or Z or a mixture thereof.

Other examples include compounds with the absolute sterochemistry as shown in formula 3: 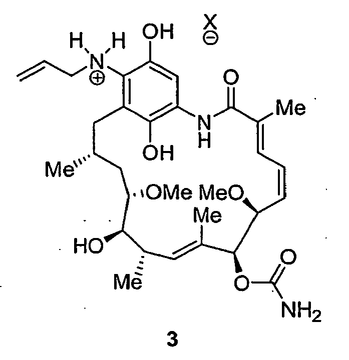 wherein X⁻ is selected from the group consisting of chloride, bromide, iodide, H₂PO₄ ⁻, HSO₄⁻, methylsulfonate, benzenesulfonate, *p*-toluenesulfonate, trifluoromethylsulfonate, and 10-camphorsulfonate, naphthalene-1-sulfonic acid-5-sulfonate, ethan-1-sulfonic acid-2-sulfonate, cyclamic acid salt, thiocyanic acid salt, naphthalene-2-sulfonate, and oxalate.

In certain embodiments, the present invention relates to the aforementioned compound and the attendant definitions, wherein X⁻ is chloride.

In certain embodiments, the present invention relates to the aforementioned compound and the attendant definitions, wherein X⁻ is bromide.

In one embodiment the present invention provides a compound of formula **4**: 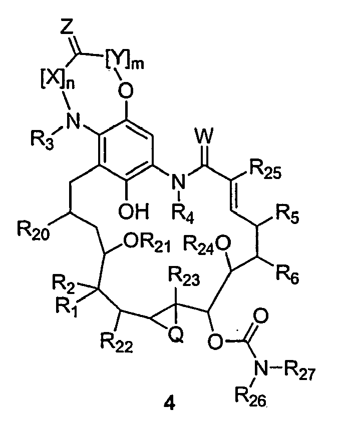 or a pharmaceutically acceptable salt thereof;
wherein, independently for each occurrence,
W is oxygen or sulfur;
Z is oxygen or sulfur;
Q is oxygen, NR, N(acyl) or a bond;
n is equal to 0, 1, or 2;
m is equal to 0, 1, or 2;
X and Y are independently C(R₃₀)₂; wherein R₃₀ for each occurrence is independently selected from the group consisting of hydrogen, alkyl, cycloalkyl, heterocycloalkyl, aralkyl, heteroaryl, and heteroaralkyl; or -[(CR₂)ₚ]-R₁₆;
R for each occurrence is independently selected from the group consisting of hydrogen, alkyl, cycloalkyl, heterocycloalkyl, aralkyl, heteroaryl, and heteroaralkyl;
R₁ is hydroxyl, alkoxyl, -OC(O)R₈, -OC(O)OR₉, -OC(O)NR₁₀R₁₁, -OSO₂R₁₂, -OC(O)NHSO₂NR₁₃R₁₄, N R₁₃R₁₄, or halide; and R₂ is hydrogen, alkyl, or aralkyl; or R₁ and R₂ taken together, along with the carbon to which they are bonded, represent -(C=O)-, - (C=N-OR)-, -(C=N-NHR)-, or -(C=N-R)-;
R₃ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ]-R₁₆;
R₄ is selected from the group consisting of H, alkyl, aralkyl, and a group having the Formula **4a**: 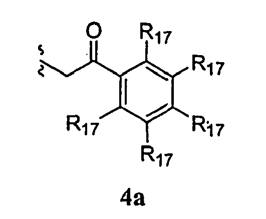
   wherein R₁₇ is selected independently from the group consisting of hydrogen, halide, hydroxyl, alkoxy, aryloxy, acyloxy, amino, alkylamino, arylamino, acylamino, aralkylamino, nitro, acylthio, carboxamide, carboxyl, nitrile, -COR₁₈, -CO₂R₁₈, ₋N(R₁₈)CO₂R₁₉, -OC(O)N(R₁₈)(R₁₉), -N(R₁₈)SO₂R₁₉, -N(R₁₈)C(O)N(R₁₈)(R₁₉), and -CH₂O-heterocyclyl;
   R₅ and R₆ are both hydrogen; or R₅ and R₆ taken together form a bond;
   R₈ is hydrogen, alkyl, alkenyl, alkynyl, aryl, cycloalkyl, heterocycloalkyl, aralkyl, heteroaryl, heteroaralkyl, or -[(CR₂)ₚ]-R₁₆;
   R₉ is alkyl, alkenyl, alkynyl, aryl, cycloalkyl, heterocycloalkyl, aralkyl, heteroaryl, heteroaralkyl, or -[(CR₂)ₚ]-R₁₆;
   R₁₀ and R₁₁ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ]-R₁₆; or-R₁₀ and R₁₁ taken together with the nitrogen to which they are bonded represent a 4-8 membered optionally substituted heterocyclic ring;
   R₁₂ is alkyl, alkenyl, alkynyl, aryl, cycloalkyl, heterocycloalkyl, aralkyl, heteroaryl, heteroaralkyl, or -[(CR₂)ₚ]-R₁₆;
   R₁₃ and R₁₄ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ]-R₁₆; or R₁₃ and R₁₄ taken together with the nitrogen to which they are bonded represent a 4-8 membered optionally substituted heterocyclic ring;
   R₁₆ for each occurrence is independently selected from the group consisting of hydrogen, hydroxyl, acylamino, -N(R₁₈)COR₁₉, -N(R₁₈)C(O)OR₁₉, -N(R₁₈)SO₂(R₁₉), -CON(R₁₈)(R₁₉), -OC(O)N(R₁₈)CR₁₉), -SO₂N(R₁₈)(R₁₉), -N(R₁₈)(R₁₉), -OC(O)OR₁₈, -COOR₁₈, -C(O)N(OH)(R₁₈), -OS(O)₂OR₁₈, -S(O)₂OR₁₈, -OP(O)(OR₁₈)(OR₁₉), -N(R₁₈)P(O)(OR₁₈)(OR₁₉), and -P(O)(OR₁₈)(OR₁₉);
   pis 1, 2, 3, 4, 5, or 6;
   R₁₈ for each occurrence is independently selected from the group consisting of hydrogen, alkyl, aryl, cycloalkyl, heterocycloalkyl, aralkyl, heteroaryl, and heteroaralkyl;
   R₁₉ for each occurrence is independently selected from the group consisting of hydrogen, alkyl, aryl, cycloalkyl, heterocycloalkyl, aralkyl, heteroaryl, and heteroaralkyl; or R₁₈ taken together with R₁₉ represent a 4-8 membered optionally substituted ring;
   R₂₀, R₂₁, R₂₂, R₂₄, and R₂₅, for each occurrence are independently alkyl;
   R₂₃ is alkyl, -CH₂OH, -CHO, -COOR₁₈, or -CH(OR₁₈)₂;
   R₂₆ and R₂₇ for each occurrence are independently selected from the group consisting of hydrogen, alkyl, aryl, cycloalkyl, heterocycloalkyl, aralkyl, heteroaryl, and heteroaralkyl; and
   the absolute stereochemistry at a stereogenic center of formula **4** may be R or S or a mixture thereof and the stereochemistry of a double bond may be E or Z or a mixture thereof.

Specific examples include the compounds shown below:

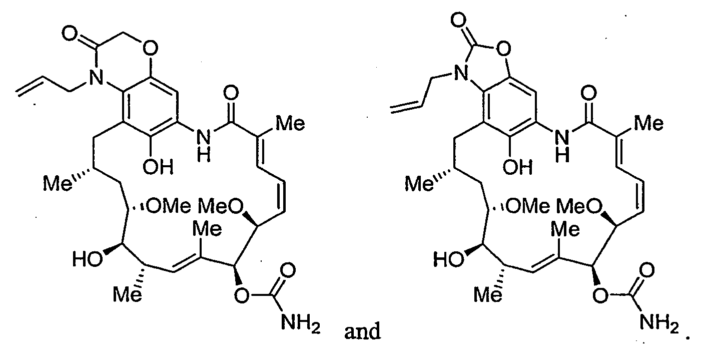

The embodiments described above and in the following sections encompass hydroquinone analogs of the geldanamycin family of molecules. In addition to reduced forms of 17-AAG (17-allylamino-18,21-dihydro-17-demethoxygeldanamycin), other preferred compounds disclosed herein relates to 18,21-dihydro-geldanamycin family including, but not limited to, 18,21-dihydro analogs of 17-Amino-4,5-dihydro-17-demethoxy-geldanamycin; 17-Methylamino-4,5-dihydro-17-demethoxygeldanamycin; 17-Cyclopropylamino-4,5-dihydro-17-demethoxygeldanamycin; 17-(2'-Hydroxyethylamino)-4,5-dihydro-17-demethoxygelclanamycin; 17-(2-Methoxyethylamino)-4,5-dihydro-17-demethoxygeldanamycin; 17-(2'-Fluoroethylamino)-4,5-dihydro-17-demethoxygeldanamycin; 17-(S)-(+)-2-Hydroxypropylamino-4,5-dihydro-17-demethoxygeldanamycin; 17-Azetidin-1-yl-4,5-dihydro-17-demethoxygeldanamycin; 17-(3-Hydroxyazetidin-1-yl)-4,5-dihydro-17-demethoxygeldanamycin; 17-Azetidin-1-yl-4,5-dihydro-11-alpha-fluoro-17-demethoxygeldanamycin; 17-(2'-Cyanoethylamino)-17-demethoxygeldanamycin; 17-(2'-Fluoroethylamino)-17-demethoxygeldanamycin; 17-Amino-22-(2'-methoxyphenacyl)-17-demethoxygeldanamycin; 17-Amino-22-(3'-methoxyphenacyl)-17-demethoxygeldanetmycin; 17-Amino-22-(4'-chlorophenacyl)-17-demethoxygeldanamycin; 17-Amino-22-(3',4'-dichlorophenacyl)-17-demethoxygeldanamycin; 17-Amino-22-(4'-amino-3'-iodophenacyl)-17-demethoxygeldanamycin; 17-Amino-22-(4'-azido-3'-iodophenacyl)-17-demethoxygeldanamycin; 17-Amino-11-alpha-fluoro-17-demethoxygeldanamycin; 17-Allylamino-11-alpha-fluoro-17-demethoxygeldanamycin; 17-Propargylamino-11-alpha-fluoro-17-demethoxygeldanamycin; 17-(2'-Fluoroethylamino)-11-alpha-fluoro-17-demethoxygeldanamycin; 17-Azetidin-1-yl-11-(4'-azidophenyl)sulfamylcarbonyl-17-demethoxygeldanamycin; 17-(2'-Fluoroethylamino)-11-keto-17-demethoxygeldanamycin; 17-Azetidin-1-yl-11-keto-17-demethoxygeldanamycin; and 17-(3'-Hydroxyazetidin-1-yl)-11-keto-17-demethoxygeldanamycin.

The compositions disclosed herein exists as salts of the reduced ansamycin, e.g., HCl or H₂SO₄ salts. In another embodiment the compounds are co-crystallized with another salt, such as an amino acid, e.g., glycine. In general, in these embodiments, the ratio of amino acid to ansamycin can vary, but is preferably from 2:1 to 1:2 amino acid:ansamycin.

### Methods of Making

A variety of methodologies can be adapted for generating the compounds disclosed herein. In general, the steps involve (1) converting the ansamycin to a 17-demethoxy-17-amino analog (e.g.; 17-AAG), (2) reducing the benzoquinone in the ansamycin to give a hydroquinone, and (3) treating said hydroquinone with a Bronsted acid. Further methodologies can be found in WO 2005/063714.

A benzoquinone-containing macrocyclic molecule, can be obtained via fermentation of a strain producing the compound (for example, see WO 03/072794 and U.S. Patent 3,595,955). Alternatively, synthetic or semi-synthetic methodology can be used to produce the ansamycin (see U.S. Patent 5,387,584 and WO 00/03737). Further, there are commercial suppliers of isolated fermentation materials, such as geldanamycin; therefore, such materials are readily available.

In preferred embodiments, synthetic methodology is used to create analogs of a natural product isolated from an organism using known methods. For example, geldanamycin is isolated from a fermentation culture of an appropriate micro-organism and may be derivatized using a variety of functionalization reactions known in the art. Representative examples include metal-catalyzed coupling reactions, oxidations, reductions, reactions with nucleophiles, reactions with electrophiles, pericyclic reactions, installation of protecting groups, removal of protecting groups, and the like. Many methods are known in the art for generating analogs of the various benzoquinone ansamycins (for examples, see U.S. Pat. Nos. 4,261,989; 5,387,584; and 5,932,566 and J. Med. Chem. 1995, 38, 3806-3812, herein incorporated by reference). These analogs are readily reduced, using methods outlined below, to yield the 18,21-dihydro derivatives disclosed herein.

Once the starting material is obtained, the benzoquinone is reduced to form a hydroquinone and then reacted with an acid; for instance HCl, to generate a C-17 ammonium hydroquinone ansamycin in an air-stable salt form. In an alternate embodiment the hydroquinone free base is reacted with an acid halide of an amino acid in place of a Bronsted acid to generate air-stable C-17 ammonium hydroquinone ansamycin co-salt derivatives. This method is exemplified in Example 3.

A variety of methods and reaction conditions can be used to reduce the benzoquinone portion of the ansamycin. Sodium hydrosulfite may be used as the reducing agent. Other reducing agents that can be used include, but are not limited to, zinc dust with acetic anhydride or acetic acid, ascorbic acid and electrochemical reductions.

Reduction of the benzoquinone moiety of the ansamycin derivative may be accomplished using sodium hydrosulfite in a biphasic reaction mixture. Typically, the geldanamycin analog is dissolved in an organic solvent, such as EtOAc. Other solvents that can be used include, but are not limited to, dichloromethane, chloroform, dichloroethane, chlorobenzene, THF, MeTHF, diethyl ether, diglyme, 1,2-dimethoxyethane, MTBE, THP, dioxane, 2-ethoxybutane, methyl butyl ether, methyl acetate, 2-butanone, water and mixtures thereof. Two or more equivalents of sodium hydrosulfite are then added as a solution in water (5-30% (m/v), preferably 10% (m/v)), to the reaction vessel at room temperature. Aqueous solutions of sodium hydrosulfite are unstable and therefore need to be freshly prepared just prior to use. Vigorous mixing of the biphasic mixture ensures reasonable reaction rates.

The reaction can readily be followed at this step by visual inspection since the starting material 17-AAG has a purple color which will disappear as the reaction proceeds to the product dihydro-17AAG, which is yellow. However, HPLC/LTV or other analytical methods can be used to monitor the reaction.

Upon completion of the reduction, the crude reaction mixture product may be used in the next step without purification to minimize oxidation of the hydroquinone. However, purification, preferably by recrystallization, can be performed if the conditions are monitored to maintain the reduced form of the benzoquinone.

The hydroquinone-containing ansamyacin is unstable and, in the presence of small amounts of oxygen or other oxidants, the hydroquinone moiety may be rapidly oxidized to the quinone species. Remarkably, the hydroquinone can be converted into an air-stable species by reaction with an acid, or by reaction with an acid halide of an amino acid. In the examples, the C-17 allyl amino group is protonated to generate a variety of air-stable C-17 ammonium salt hydroquinone geldanamycin analogs. In addition, the C-17 ammonium salt hydroquinones formed have the added benefit of being highly soluble in aqueous solutions (>200 mg/mL), unlike 17-AAG (<100 µg/mL).

The ammonium salt hydroquinone is formed by the addition of a solution of an acid, such as HCl, in an organic solvent, such as EtOAc, DCM, IPA or dioxane, to the hydroquinone containing ansamycin in an organic solution; the organic solvents may be independently acetone, dichloromethane, chloroform, dichloroethane, chlorobenzene, THF, MeTHF, diethyl ether, diglyme, 1,2-dimethoxyethane, MTBE, THP, dioxane, 2-ethoxybutane, methyl butyl ether, methyl acetate, 2-butanone, under nitrogen.

The ammonium salt of the hydroquinone is collected by filtration in cases where the product precipitates from solution. In cases where the ammonium salt hydroquinone does not precipitate, the reaction solution is concentrated under reduced pressure to yield the product.

A variety of air-stable ammonium salt hydroquinone ansamycins can be synthesized by using organic or inorganic acids. Some acids that can be used include, but are not limited to HCl, HBr, H₂SO₄, methansulfonic acid, benzenesulfonic acid, p-toluenesulfonic acid, triflic acid, camphorsulfonic acid, naphthalene-1,5-disulfonic acid, ethan-1,2-disulfonic acid, cyclamic acid, thiocyanic acid, naphthalene-2-sulfonic acid, oxalic acid, and the like. See, for example, Berge et al. (1977) "Pharmaceutical Salts", J. Pharm. Sci. 66:1-19. The acid used preferably should have a pKa sufficient to protonate the aniline nitrogen. Thus, any acid with a pKa between about -10 and about 7, preferably about -10 and about 4, more preferably between about -10 and about 1, and even more preferably between about -10 and about -3 may be used to generate the ammonium salt hydroquinone.

Recrystallization is accompished by dissolving the compound in the minimal amount of an inert polar organic solvent, such as MeOH, EtOH, or IPA, and slowly adding a miscible organic solvent, such as an aliphatic ether, ethyl acetate, methyl acetate, chloroform or DCM, causing the solution to become turbid. The mixture is then allowed to sit for a suitable period of time, and optionally cooled, and the resulting solid is collected by filtration, washed and dried under reduced pressure.

### Pharmaceutical Compositions

When the compounds of the Formula **1** and **3** and their pharmaceutically acceptable salts are used as antiproliferative agents, such as anticancer agents, they can be administered to a mammalian subject either alone or in combination with pharmaceutically acceptable carriers or diluents in a pharmaceutical composition according to standard pharmaceutical practice.

As set out above, certain embodiments of the present compounds may contain a basic functional group, such as amino or alkylamino, and are, thus, capable of forming pharmaceutically-acceptable salts with pharmaceutically-acceptable acids. The term "pharmaceutically-acceptable salts" in this respect, refers to the relatively non-toxic, inorganic and organic acid addition salts of compounds of the present invention. These salts can be prepared *in situ* in the administration vehicle or the dosage form manufacturing process, or by separately reacting a purified compound of the invention in its free base form with a suitable organic or inorganic acid, and isolating the salt thus formed during subsequent purification. Representative salts include the hydrobromide, hydrochloride, sulfate, bisulfate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, for example, Berge et al. (1977) "Pharmaceutical Salts", J. Pharm. Sci. 66:1-19)

The pharmaceutically acceptable salts of the compounds disclosed herein include the conventional nontoxic salts or quaternary ammonium salts of the compounds, e.g., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In other cases, the compounds disclosed herein may contain one or more acidic functional groups and, thus, are capable of forming pharmaceutically-acceptable salts with pharmaceutically-acceptable bases. The term "pharmaceutically-acceptable salts" in these instances refers to the relatively non-toxic, inorganic and organic base addition salts of compounds disclosed herein. These salts can likewise be prepared *in situ* in the administration vehicle or the dosage form manufacturing process, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, for example, Berge *et al., supra*)

Wetting agents, emulsifiers and lubricants, such as sodium lauryl sulfate and magnesium stearate, as well as coloring agents, release agents, coating agents, sweetening, flavoring and perfuming agents, preservatives, solubilizing agents, buffers and antioxidants can also be present in the compositions.

Examples of pharmaceutically-acceptable antioxidants include, but are not limited to: (1) water soluble antioxidants, such as ascorbic acid, cysteine hydrochloride, sodium bisulfate, sodium metabisulfite, sodium sulfite, thioglycerol, sodium mercaptoacetate, and sodium formaldehyde sulfoxylate; (2) oil-soluble antioxidants, such as ascorbyl palmitate, butylated hydroxyanisole (BHA), butylated hydroxytoluene (BHT), lecithin, propyl gallate, alpha-tocopherol.

Examples of pharmaceutically-acceptable buffering agents include, but are not limited to citrate, ascorbate, phosphate, bicarbonate, carbonate, fumarate, acetate, tartarate and malate.

Examples of pharmaceutically-acceptable solubilizing agents include, but are not limited to polyoxyethylene sorbitan fatty acid esters (including polysorbate 80), polyoxyethylene stearates, benzyl alcohol, ethyl alcohol, polyethylene glycols, propylene glycol, glycerin, cyclodextrin, and poloxamers.

Examples of pharmaceutically-acceptable complexing agents include, but are not limited to, cyclodextrins (alpha, beta, gamma), especially substituted beta cyclodextrins such as 2-hydroxypropyl-beta, dimethyl beta, 2-hydroxyethyl beta, 3-hydroxypropyl beta, trimethyl beta.

Examples of pharmaceutically-acceptable metal chelating agents include, but are not limited to, citric acid, ethylenediamine tetraacetic acid (EDTA) and its salt, DTPA (diethylene-triamine-penta-acetic acid) and its salt, EGTA and its salt, NTA (nitriloacetic acid) and its salt, sorbitol and its salt, tartaric acid and its salt, N-hydroxy iminodiacetate and its salt, hydroxyethyl-ethylene diamine-tetraacetic acid and its salt, 1- and 3-propanediamine tetra acetic acid and their salts, 1- and 3-diamino-2-hydroxy propane tetra-acetic acid and their salts, sodium gluconate, hydroxy ethane diphosphonic acid and its salt, and phosphoric acid and its salt.

Pharmaceutical compositions disclosed herein suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, chelating agents, solutes which render the formulation isotonic with the blood of the intended recipient or suspending or thickening agents. In the examples, the active ingredients are brought together with the pharmaceutically acceptable carriers in solution and then lyophilized to yield a dry powder. The dry powder is packaged in unit dosage form and then reconstituted for parental administration by adding a sterile solution, such as water or normal saline, to the powder.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the compounds disclosed herein may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

It is important for formulations of the compounds disclosed herein to provide solubility and redox stability to this hydroquinone salt. Compounds disclosed herein are significantly solubilized at lower pH when the amine is protonated. The species distribution is important since the ionized form is more soluble while the free base (unionized form) is less soluble. Therefore a formulation will optimize the solubility by controlling the pH of the solution. A buffering agent such as citrate which has a high buffering capacity at a preferred pH range is one such preferred formulation component. Preferably buffering agents will buffer the formulation between a pH of about 1.5 to about 5.0, more preferably between a pH of about 1.8 to about 3.5, and even more preferably between a pH of about 3 to about 3.3.

The hydoquinone analogs disclosed herein may oxidize on prolonged standing in solution. Heavy metals, such as iron and copper, are capable of catalyzing oxidation reactions and can be found in trace quantities in typical reagents and labware. Protection from the oxidizing nature of heavy metals can be afforded by metal chelators such as EDTA (ethylene diamine tetraacetic acid). Other known chelators are, for example, citric acid, DTPA (diethylene-triamine-penta-acetic acid) and its salt, EGTA and its salt, NTA (nitriloacetic acid) and its salt, sorbitol and its salt, tartaric acid and its salt, N-hydroxy iminodiacetate and its salt, hydroxyethyl-ethylene diamine-tetraacetic acid and its salt, 1-and 3-propanediamine tetra acetic acid and their salts, 1- and 3-diamino-2-hydroxy propane tetra-acetic acid and their salts, sodium gluconate, hydroxy ethane diphosphonic acid and its salt, and phosphoric acid and its salt.

Another important method of preventing oxidation is to add an anti-oxidant. One preferred anti-oxidant is ascorbic acid (ascorbate). This reagent protects compounds from the oxidizing effect of molecular oxygen dissolved in aqueous media. In certain embodiments, ascorbate is used as a component in formulations of the hydroquinone analogs disclosed herein.

### Methods of Therapy and Treatment

The hydroquinone ansamycins disclosed herein are water soluble, and can therefore be administered to subjects in aqueous solutions. These compounds rapidly oxidize to 17-amino substituted benzoquinone geldanamycin analogs (e.g., 17-AAG) *in vitro* and *in vivo* at physiological pH. For example, Compound 2 and 17-AAG interconvert at physiologic conditions. As such, the hydroquinone ansamycins exhibit similar biological activities and therapeutic profiles as 17-amino substituted geldanamycin analogs and may be used for the same therapeutic indications that 17-amino substituted geldanamycin analogs are useful in treating. 17-amino substituted geldanamycin analogs, and in particular 17-AAG, are highly potent and selective inhibitors of Hsp90.

The invention provides methods for treating, ameliorating one or more of the symptoms of, and reducing the severity of hyperproliferative disorders, e.g., cancer, as well as other Hsp90 mediated disorders or conditions. The methods of the present invention involve administering a therapeutically effective amount of a hydroquinone ansamycin to a subject suffering from any of these conditions.

Examples of hyperproliferative disorders that can be treated with the hydroquinone ansamycins described herein include cancers of the hematopoietic system, immune system, endocrine system, pulmonary system, gastrointestinal system, musculoskeletal system, hepato-biliary system, reproductive system, central nervous system, or urologic system.

Also included are cancers located in the myeloid cells, lymphoid tissues, pancreas, thyroid, lungs, small intesting, colon, rectum, anue, liver, skin, bone, ovaries, uterus, cervix, breast, prostate, testicles, brain, brainstem, meninges, kidney, or bladder.

Further included are breast cancer, multiple myeloma (MM), prostate cancer, Hodgkin lymphoma, non-Hodgkin lymphoma, acute lymphocytic leukemia (ALL), chronic lymphocytic leukemia (CLL), acute myeloid leukemia, chronic myeloid leukemia (CML), renal cell carcinoma, malignant melanoma, pancreatic cancer, gastric cancer, esophageal cancer, lung cancer (e.g., small cell lung cancer or non small cell lung cancer), colorectal carcinoma, colon cancer, brain cancer, renal cancer, hepatocellular cancer, head and neck cancer, bladder cancer, thyroid cancer, ovarian cancer, cervical cancer, or myelodysplastic syndrome.

Myeloproliferative disorders, including essential thrombocythemia, agnogenic myeloid metaplasia, polycythemia vera, and hypereosinophilic syndrome (HES) are also included.

Systemic mastocytosis, dermatofibrosarcoma protuberans, synovial carcinoma, Ewing's sarcoma, chronic myelomonocytic leukemia, chronic myelogenous leukemia, familiar hypereosinophilia, chronic eosinophilic leukemia, thyroid cancer, Adenoid cystic carcinoma of the head and neck, thymic carcinoma, gastric adenocarcinoma, and chordoma are further included.

The present invention provides methods for treating hyperproliferative disorders associated with aberrations in signal transduction pathways mediated by fusion proteins or ligand independent receptor tyrosine kinases. For example, Gorre et al. have shown that inhibition of Hsp90 can induce degradation of mutant Bcr-Abl fusion proteins. Blood (2002) 100:3041-44. Hematopoietic cells expressing two mutant Bcr-Abl fusion proteins found in imatinib mesylate-resistant patients (harbouring T315I or E255K mutations) were derived and tested for sensitivity to 17-AAG. This compound induced the degradation of wild-type and mutant Bcr-Abl proteins as well as inhibited cell growth, which suggests that the hydroquinone ansamycins of the present invention can be used to treat cancers, such as CML, associated with fusion proteins, including those with point mutations.

Shen et al. have shown that 17-AAG blocks hepatocyte growth factor/scatter factor-mediated c-Met tyrosine kinase receptor *(*Bioorg. Med. Chem. (2005) 13: 4960-71), which suggests the utility of the hydroquinone ansamycins described herein in treating hyperproliferative disorders associated with aberrations in signal transduction pathways mediated by the fusion protein Tpr-Met, such as gastric cancer and small cell lung cancer.

Marsee et al. have shown that inhibition of Hsp90 function with 17-AAG reduces RET/PTC1 (a rearranged form of the RET tyrosine kinase commonly seen in papillary thyroid carcinomas) protein levels. J. Biol. Chem. (2004) 279:43990-7. This suggests that the hydroquinone ansamysins disclosed herein can be used to treat cancers associated with aberration in a signal transduction pathway mediated by RET, such as thyroid cancer.

Yang et al. have shown that the fusion protein RUNX1-ETO promotes the expansion of hematopoietic stem/progenitor cells and induces leukemia in associaton with other genetic alterations. 17-AAG was shown to trigger the degradation of this protein. Oncogene (2006) 1-11. This suggests that the hydroquinone ansamysins disclosed herein can be used to treat hyperproliferative disorders associated with aberrations in the signal transduction pathway mediated by this protein.

The invention also provides methods for treating hyperproliferative disorders associated with aberrations in signal transduction pathways mediated by mutant gain-of function proteins, such as c-Kit. For example, Fumo et al. have shown that treatment with 17-AAG of the mast cell line HMC-1.2, harboring the Asp816Val and Va1560Gly Kit mutations, and the cell line HMC-1.1, harboring a single Va1560Gly mutation, causes the level and activity of Kit to be down-regulated and promotes cell death in both cell lines. In addition, neoplastic mast cells isolated from patients with mastocytosis, incubated with 17-AAG ex vivo, are sensitive to 17-AAG. Blood (2004) 103: 1078-84. These data suggest that the hydroquinone ansamycins disclosed herein may be effective in the treatment of c-Kit-related diseases, including mastocytosis, gastrointestinal stromal tumors (GISTs), mast cell leukemia, subtypes of acute myelogenous leukemia, and testicular cancer.

Inhibition of Hsp90 by Compound 2 was evaluated in imatinib mesylate (IM)-sensitive (GIST882) and IM-resistant GIST (GIST430, GIST48, GIST62) cell lines that are characterized by secondary kinase domain mutations (GIST48 and GIST430) and by loss of Kit expression (GIST62). Biological consequences of Hsp90 inhibition were determined by immunoblotting for Kit signaling pathways and by cell proliferation and apoptosis assays. Compound 2 inhibited IM-sensitive and IM-resistant KIT oncoproteins: IC50s for phospho and total Kit were 150nM and 220nM in GIST882, 160nM and 200nM in GIST48, and 100nM and 70nM in GIST430. Inhibition of downstream signaling intermediates AKT and S6 was seen in the KIT-positive GISTs (GIST882, GIST430 and GIST48) but not in the KIT-negative GIST62, suggesting that the effects of Compound 2 depend on the Kit target. Likewise, inhibition of cell proliferation by Compound 2 (500 nM) was seen in GIST48 and GIST430 (88% and 34% inhibition, respectively) but not in GIST62 (12% inhibition). Compound 2 (>100nM) induced apoptosis in the Kit-positive GISTs. These results indicate that Hsp90 inhibition by Compound 2 has strong antiproliferative and proapoptotic effects in IM-resistant GISTs at clinically achievable doses. Bauer et al., poster presented at EORTC (2005).

FMS-like TK-3 ("FLT-3"), a member of the class III receptor tyrosine kinases, is a known client protein of Hsp90. Treatment with 17-AAG was shown to disrupt the chaperone association of Hsp90 with FLT-3, directing it to polyubiquitylation and proteasomal degradation. Blood (2005) 105:1768-76.

It has been shown that the presence of the activating internal tandem duplication mutation (ITD) in the juxtamembrane domain or a point mutation in the kinase domain of the FMS-like tyrosine kinase-3 (FLT-3) mediates ligand-independent growth and survival signaling in approximately one-third of acute myelogenous leukemia (AML) patients. In addition, previous studies demonstrated that treatment of human AML MV411 cells (containing an ITD FLT 3) with 17-AAG attenuated the levels of FLT-3 by inhibiting its association with the Hsp90 chaperone and stimulating the poly-ubiquitination and proteasomal degradation of FLT-3.

MV4 11 cells were treated with increasing concentrations of Compound 2 or 17-AAG for 3 days. Both compounds were cytotoxic, with an IC50 of approximately 30 nM. Phosphorylated ITD FLT-3 expression levels are also inhibited in these cells with an apparent IC50 of 50 nM at 24 hours. There is good correlation between FLT-3 degradation and cytotoxicity by both 17-AAG and Compound 2 in MV411 cells. By contrast, another AML cell line, KG 1, which expresses the wild-type form of the FLT-3 receptor, exhibited less sensitivity toward Compound 2 with regard to cytotoxicity (72 h) and degradation (24 h) of the wild-type phosphorylated FLT-3 protein. These data indicate that mutated FLT-3 is more dependent on Hsp90 function than wild-type FLT 3. These data suggest a role for the hydroquinone ansamycins disclosed herein for treating conditions, such as AML, associated with an aberration in a signal transduction pathway mediated by FLT-3.

The invention further provides a method for treating cancers, such as non small cell lung cancer (NSCLC), associated with the epidermal growth factor receptor ("EGFR"). Shimamura et al. have shown that mutant EGFR proteins found in NSCLC are client proteins of Hsp90 and are degraded following Hsp90 inhibition. Mutant EGFR expression was depleted after only 4 hours of exposure to geldanamycin, whereas diminution of wild-type EGFR was less substantial and seen only following 12 hours of exposure. Cancer Research (2005) 65:6401-6408. These data suggest that Hsp90 inhibition, for example using the hydroquinone ansamycins compounds disclosed herein, can be used for the treatment of cancers such as EGFR-mutant NSCLC, and other conditions associated with an aberration in the signal transduction pathway mediated by EGFR.

The invention provides a method for treating cancers, such as CLL, associated with the zeta-associated protein of 70 kDa (ZAP-70). Castro et al. have shown that ZAP-70+ CLL cells expressed activated heat-shock protein 90 (Hsp90) with high binding affinity for Hsp90 inhibitors, such as 17-AAG, whereas normal lymphocytes or ZAP-70- CLL cells expressed nonactivated Hsp90. Blood (2005) 106: 2506-2512. Treatment with 17-AAG induced ZAP-70 degradation and apoptosis in CLL cells but not in T cells, and also impaired B-cell receptor signaling in leukemia cells. This suggests that Hsp90 inhibitors, such as the hydroquinone ansamycins disclosed herein, could be valuable therapeutically in patients with aggressive CLL and other conditions associated with ZAP-70.

The invention further provides a method for treating cancers, such as breast or lung cancer, associated with Her2/Erb2, and well as other conditions associated with an aberration in the signal transduction pathway mediated by Her2/Erb2. As an illustrative example, de Candia et al. treated mice with 17-AAG and demonstrated the effectiveness of 17-AAG in treating Her2/neu-dependent breast tumors. PNAS (2003) 100:12337-12342.

Jerome et al. have demonstrated the role of Hsp90 inhibitors in stabilizing growth factor receptors such as the PDGF receptor. Growth Factors (1991) 4:317-27. This suggests the utility of the hydroquinone ansamycins described herein in treating cancers, such as HES, and other conditions associated with an aberration in the signal transduction pathway mediated by the PDGF receptor.

The invention provides a method for treating cancers such as prostate cancer, associated with Akt (where the tumor suppressor status of PTEN is PTEN-/- or PTEN+/-). Georgakis et al. have shown that inhibition of Hsp90 function by 17-AAG in Hodgkin's lymphoma cells down-regulates Akt kinase, dephosphorylates extracellular signal-regulated kinase, and induces cell cycle arrest and cell death. Clin. Cancer Res. (2006) 12:584-90. This suggests the utility of the hydroquinone ansamycins described herein in treating prostate cancer and Hodgkin's lymphoma and other conditions associated with Akt.

Mutations in the androgen receptor (AR) may account, in part, for prostate cancer progression after castration or treatment with anti-androgens. 17-AAG has been shown to stimulate the rapid degradation of the AR in prostate cancer cells. Clin. Cancer Res. (2002) 8:986-993.

LnCAP, a PSA-secreting prostate cancer cell line expressing a mutated AR, was incubated with increasing concentrations of Compound 2. The level of AR protein and the cytotoxic response was monitored. Alamar Blue studies demonstrated that Comound 22 was cytotoxic for LnCAP cells with an EC₅₀ of ∼75 nM. A Western blot experiments showed that the AR protein was significantly reduced with > 30 nM of Compound 2 24 hours after treatment. These data demonstrate that the AR is significantly degraded in response to Compound 2 treatment and this correlates with cell killing by Compound 2. This suggests that the hydroquinone ansamycin compounds disclosed herein could be useful in the inhibition of prostate cell growth and survival. They can also be used to treat conditions associated with aberrations in the signal transduction pathway mediated by the estrogen receptor, the progesterone receptor, or the glucocorticoid receptor.

The invention provides a method for treating cancers, such as melanoma, associated with an aberration in a signal transduction pathway mediated by B-Raf. Grbovic et al. have shown that exposure of melanoma cells and tumors to 17-AAG results in the degradation of mutant B-Raf, inhibition of mitogen-activated protein kinase activation and cell proliferation, induction of apoptosis, and antitumor activity. PNAS (2006) 103:57-62. This suggests that inhibiting Hsp90 using, for example, the hydroquinone ansamycins disclosed herein, represents a therapeutic strategy for the treatment of melanoma.

Mitsiades et al. have shown that Hsp90 inhibitors simultaneously suppresses in MM cells the expression and/or function of multiple levels of signaling effectors such as IKK and NF-kappaB. Blood (2006) 107:1092-100. This suggests that the hydroquinone ansamycins described herein can be used to treat hyperproliferative disorders associated with aberration of the signal transduction pathway mediated by IKK or NF-kappaB.

The hydroquinone ansamycins disclosed herein can be used to treat conditions associated with any client protein of Hsp90. Examples of such proteins include Chk1, Telomerase, Hifl α, MMP2, MET, FAK, RIP, PLK, and NPM-AL.

In addition, the present invention provides methods for treating melanoma *(See,* Anti-Cancer Drugs (2004) 15: 377-388), prostate cancer *(See,* Clin. Cancer Res. (2002) 8: 986-993), breast cancer (*See,* Cancer Res. (2001) 61: 2945-2952), non-small cell lung cancer *(See,* Ann. Thorac. Surg. (2000) 70: 1853-1860), leukemias *(See,* Cancer Res. (2001) 61: 1799-1804), and colon cancer *(See,* J. Natl. Cancer Inst. (2003) 95: 1624-1633).

### Combination Therapy

The compounds disclosed herein can be used at cytotoxic or sub-cytotoxic levels in combination with at least one other agent in order to achieve selective or improved activity in the treatment of cancer. In certain embodiments, the compounds disclosed herein are used to reduce the cellular levels of properly folded Hsp90 client proteins, which are then effectively inhibited by the second agent or whose degradation in the proteasome is inhibited using a proteasome inhibitor, e.g., Velcade^{™}. Binding of the client proteins to Hsp90 stabilizes the client proteins and maintains them in a soluble, inactive form ready to respond to activating stimuli. Binding of a hydroquinone ansamycin to Hsp90 results in targeting of the client protein to the proteasome, and subsequent degradation. Using an agent that targets and inhibits the proteasome blocks proteasome degradation leading to increased in cellular apoptosis and cell death.

Some examples of antineoplastic agents which can be used in combination with the methods of the present invention include, in general, alkylating agents; anti-angiogenic agents; anti-metabolites; epidophyllotoxin; an antineoplastic enzyme; a topoisomerase inhibitor; procarbazine; mitoxantrone; platinum coordination complexes; anti-mitotics; biological response modifiers and growth inhibitors; hormonal/anti-hormonal therapeutic agents and haematopoietic growth factors.

Exemplary classes of antineoplastic agents further include the anthracycline family of drugs, the vinca drugs, the mitomycins, the bleomycins, the cytotoxic nucleosides, the epothilones, discodermolide, the pteridine family of drugs, diynenes and the podophyllotoxins.

Particularly useful members of those classes include, for example, carminomycin, daunorubicin, aminopterin, methotrexate, methopterin, dichloromethotrexate, mitomycin C, porfiromycin, 5-fluorouracil, 6-mercaptopurine, gemcitabine, cytosine arabinoside, podophyllotoxin or podophyllotoxin derivatives such as etoposide, etoposide phosphate or teniposide, melphalan, vinblastine, vincristine, leurosidine, Velcade, doxorubicin, vindesine, leurosine, imatinib mesylate, paclitaxel, taxol, and the like. In a preferred embodiment, the antineoplastic agent is Velcade, doxorubicin, taxotere, docetaxel, paclitaxel, cis-platin, imatinib mesylate, or gemcitebine. In a preferred embodiment, the antineoplastic agent is Velcade or doxorubicin.

Other useful antineoplastic agents include estramustine, carboplatin, cyclophosphamide, bleomycin, gemcitibine, ifosamide, melphalan, hexamethyl melamine, thiotepa, cytarabin, idatrexate, trimetrexate, dacarbazine, L-asparaginase, camptothecin, CPT-11, topotecan, ara-C, bicalutamide, flutamide, leuprolide, pyridobenzoindole derivatives, interferons and interleukins.

The compounds disclosed herein can also be used in combination with kinase inhibitors, including sunitinib, gefitinib, sorafenib, trastuzumab, bevacizumab, and lapatinib.

The chemotherapeutic agent and/or radiation therapy can be administered according to therapeutic protocols well known in the art. It will be apparent to those skilled in the art that the administration of the chemotherapeutic agent and/or radiation therapy can be varied depending on the disease being treated and the known effects of the chemotherapeutic agent and/or radiation therapy on that disease. Also, in accordance with the knowledge of the skilled clinician, the therapeutic protocols (e.g., dosage amounts and times of administration) can be varied in view of the observed effects of the administered therapeutic agents (i.e., antineoplastic agent or radiation) on the patient, and in view of the observed responses of the disease to the administered therapeutic agents.

Also, in general, compounds disclosed herein and the chemotherapeutic agent do not have to be administered in the same pharmaceutical composition, and may, because of different physical and chemical characteristics, have to be administered by different routes. For example, compounds disclosed herein may be administered intravenously to generate and maintain good blood levels, while the chemotherapeutic agent may be administered orally. The determination of the mode of administration and the advisability of administration, where possible, in the same pharmaceutical composition, is well within the knowledge of the skilled clinician. The initial administration can be made according to established protocols known in the art, and then, based upon the observed effects, the dosage, modes of administration and times of administration can be modified by the skilled clinician.

The particular choice of chemotherapeutic agent or radiation will depend upon the diagnosis of the attending physicians and their judgment of the condition of the patient and the appropriate treatment protocol.

A compound disclosed herein, and chemotherapeutic agent and/or radiation may be administered concurrently (e.g., simultaneously, essentially simultaneously or within the same treatment protocol) or sequentially, depending upon the nature of the proliferative disease, the condition of the patient, and the actual choice of chemotherapeutic agent and/or radiation to be administered in conjunction (i.e., within a single treatment protocol) with a compound disclosed herein.

If a compound disclosed herein, and the chemotherapeutic agent and/or radiation are not administered simultaneously or essentially simultaneously, then the optimum order of administration of the compound disclosed herein, and the chemotherapeutic agent and/or radiation, may be different for different tumors. Thus, in certain situations the compound disclosed herein may be administered first followed by the administration of the chemotherapeutic agent and/or radiation; and in other situations the chemotherapeutic agent and/or radiation may be administered first followed by the administration of a compound disclosed herein. This alternate administration may be repeated during a single treatment protocol. The determination of the order of administration, and the number of repetitions of administration of each therapeutic agent during a treatment protocol, is well within the knowledge of the skilled physician after evaluation of the disease being treated and the condition of the patient. For example, the chemotherapeutic agent and/or radiation may be administered first, especially if it is a cytotoxic agent, and then the treatment continued with the administration of a compound disclosed herein followed, where determined advantageous, by the administration of the chemotherapeutic agent and/or radiation, and so on until the treatment protocol is complete.

Thus, in accordance with experience and knowledge, the practicing physician can modify each protocol for the administration of a component (therapeuticagent, i.e., compound disclosed herein, chemotherapeutic agent or radiation) of the treatment according to the individual patient's needs, as the treatment proceeds.

### Dosage

When the compounds disclosed herein are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

Actual dosage levels of the active ingredients in the pharmaceutical compositions disclosed herein may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound disclosed herein employed, or salt thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable dose of a compound of the invention will be that amount of the compound which is the lowest safe and effective dose to produce a therapeutic effect. Such an effective dose will generally depend upon the factors described above. Generally, intravenous doses of the compounds disclosed herein for a patient will range from about 10 mg to about 1000 mg per meter² dosed twice per week, preferably between about 75 mg to 750 mg per meter² dosed twice per week, and even more preferably 100 mg to 500 mg per meter² dosed twice per week.

While it is possible for a compound disclosed herein to be administered alone, it is preferable to administer the compound as a pharmaceutical formulation (composition).

The patient receiving this treatment is any animal in need, including primates, in particular humans, and other mammals such as equines, cattle, swine and sheep; and poultry and pets in general.

One or more other active compounds may be added to the formulations described above to provide formulations for combination cancer therapy.

The compounds can be administered parenterally; i.e., other than by enteral and topical administration. The compounds can be administered by injection, e.g., intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The compounds can also be administered systemically or peripherally; i.e., administration other than directly into the central nervous system, such that it enters the patient's system and, thus, is subject to metabolism and other like processes, for example, subcutaneous administration.

### EXEMPLIFICATION

The invention now being generally described, it will be more readily understood by reference to the following examples. Further, the amino acid are represented in zwitterionic form and can also be further protonated and exist as the salt.

### Example 1

### Preparation of Air-stable Hydroquinone Derivatives of the Geldanamycin Family or Molecules

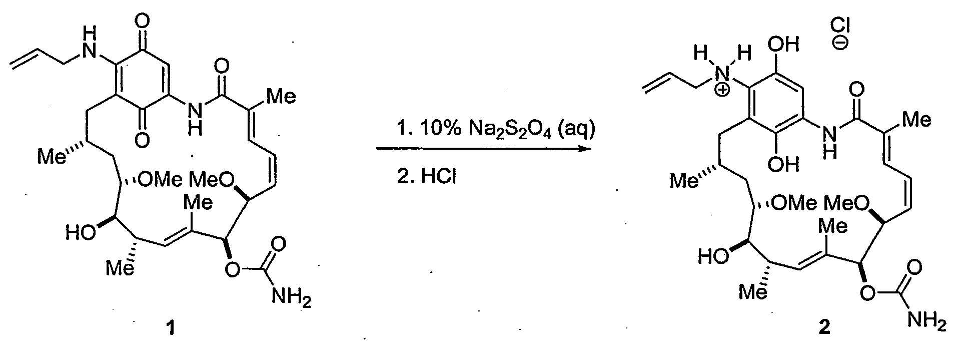

17-Allylamino-17-Demethoxygeldanamycin (10.0 g, 17.1 mmol) in ethyl acetate (200 mL) was stirred vigorously with a freshly prepared solution of 10% aqueous sodium hydrosulfite (200 mL) for 2 h at ambient temperature. The color changed from dark purple to bright yellow, indicating a complete reaction. The layers were separated and the organic phase was dried with magnesium sulfate (15 g). The drying agent was rinsed with ethyl acetate (50 mL). The combined filtrate was acidified with 1.5 M hydrogen chloride in ethyl acetate (12 mL) to pH 2 over 20 min. The resulting slurry was stirred for 1.5 h at ambient temperature. The solids were isolated by filtration, rinsed with ethyl acetate (50 mL) and dried at 40°C, 1 mm Hg, for 16 h to afford 9.9 g (91 %) of off-white solid. Crude hydroquinone hydrochloride (2.5 g) was added to a stirred solution of 5% 0.01 N aq. hydrochloric acid in methanol (5 mL). The resulting solution was clarified by filtration then diluted with acetone (70 mL). Solids appeared after 2-3 min. The resulting slurry was stirred for 3 h at ambient temperature, then for 1 h at 0-5 °C. The solids were isolated by filtration, rinsed with acetone (15 mL) and dried

### Example 2

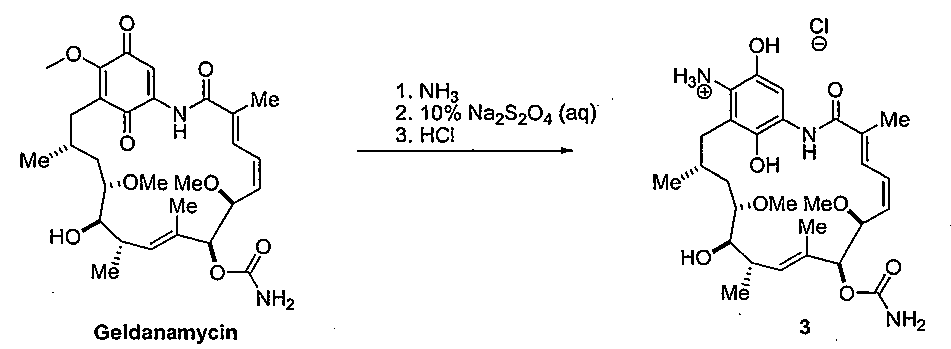

Geldanamycin (1.12g, 2 mmol, 1 equiv) was added to anhydrous DCM (5 mL). NH₃ in MeOH was added to this solution (9 mL, 100 mmol, 50 equiv) and was allowed to stir for 24 hours. At which point the reaction solution was diluted with DCM and extracted with water, followed by dilute HCl. The organic layer was collected washed with brine, dried over Na₂SO₄ and concentrated to yield a purple solid. This solid was recrystalized twice from acetone/heptanes to yield 0.239 of 17-amino-17-demethoxygeldanamycin.

17-amino-17-demethoxygeldanamycin (0.55g, 1 mmol, 1 equiv) was dissolved in EtOAc (100 mL). A freshly prepared solution of 10% aqueous sodium hydrosulfite (10 mL) was added and stirred for 1 h at ambient temperature. The color changed from dark purple to bright yellow, indicating a complete reaction. The layers were separated and the organic phase was dried with magnesium sulfate. The drying agent was rinsed with ethyl acetate (2 X 10 mL). The combined filtrate was acidified with 1.5 M hydrogen chloride in ethyl acetate (1 mL) to pH 2 over 20 min. The resulting slurry was stirred for 1.5 h at ambient temperature. The solids were isolated by filtration, rinsed with ethyl acetate (10 mL) and dried under vacuum to yield the product (0.524g, 87% yield).

### Example 3

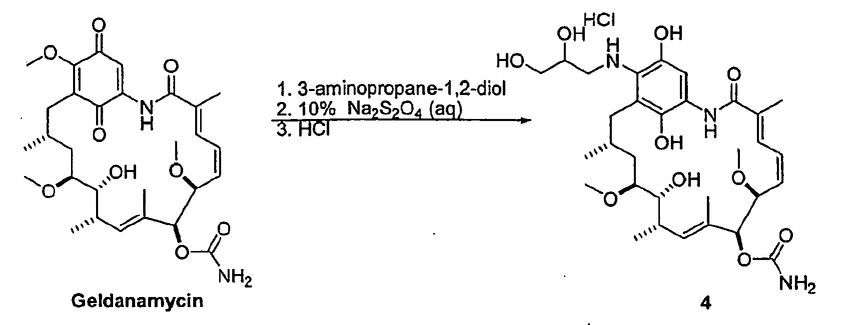

Geldanamycin (0.500g, 0.892 mmol, 1 equiv) was dissolved in THF (10 mL) 3-amino-1,2-propanediol (0.813g, 8.92 mmol, 10 equiv). The reaction was stirred for 64 hours. The reaction was then quenched with dilute HCl and extracted with EtOAc. The organic layer was collected dried over MgSO4 and concentrated under reduced pressure. The crude material was purified using column chromatography to yield 27 mg of the 17-amino substituted geldenamycin.

The 17-amino geldanmycin (.200 g, 0.323 mmol, 1 equiv) was dissolved in EtOAc (4 mL) and treated with a freshly prepared 10% solution of Na₂S₂O₄ in water (4 mL). This mixture was vigorously stirred for 1 hour. The organic layer was then collected. The aqueous layer was extracted with 2X5 mL of EtOAc. The organic layers were combined, washed with water, dried over Na₂SO₄. The organic layer was then treated with HCl in EtOAc (1.6 M, 0.6 mL) and stirred for 20 minutes. The reaction solution was concentrated under reduced pressure to yield the product (0.009 g).

### Example 4

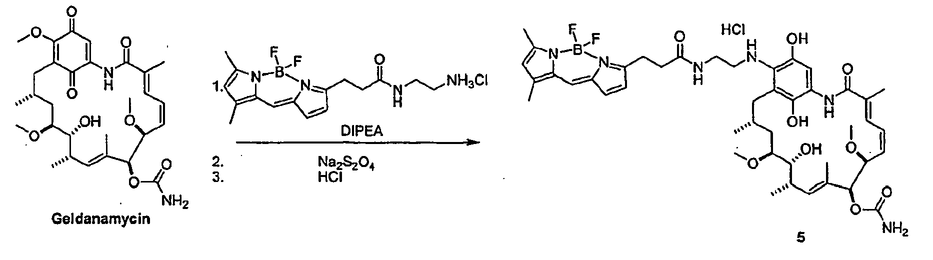

Geldanamycin (0.022g, 0.04 mmol, 1.5 equiv) and BODIPY-FL-EDA-HCl (0.010g, 0.026 mmol, 1 equiv) were added to anhydrous DCM (2mL). DIPEA (30 uL, 0.16 mmol, 6 equiv) was added and the reaction solution was stirred under nitrogen for 72 hours. The reaction was then diluted with DCM, extracted with water, dried over Na₂SO₄and concentrated under reduced pressure. The crude was purified by column chromatography to yield the 17-amino substituted benzoquinone. This material was dissolved in EtOAc (20 mL) and treated with a freshly prepared 10% solution of Na₂S₂O₄ in water (5 mL). This mixture was vigorously stirred for 1 hour. The organic layer was then collected. The aqueous layer was extracted with 2X5 mL of EtOAc. The organic layers were combined, washed with water, dried over Na₂SO₄. The organic layer was then treated with HCl in EtOAc (1.6 M, 0.6 mL) and stirred for 20 minutes. The reaction solution was then concentrated to dryness under reduce pressure. The crude was purified by reslurrying the material from EtOAc/MTBE. The solid was washed with MTBE and dried under reduced pressure to yield the product (0.04 g).

### Example 5

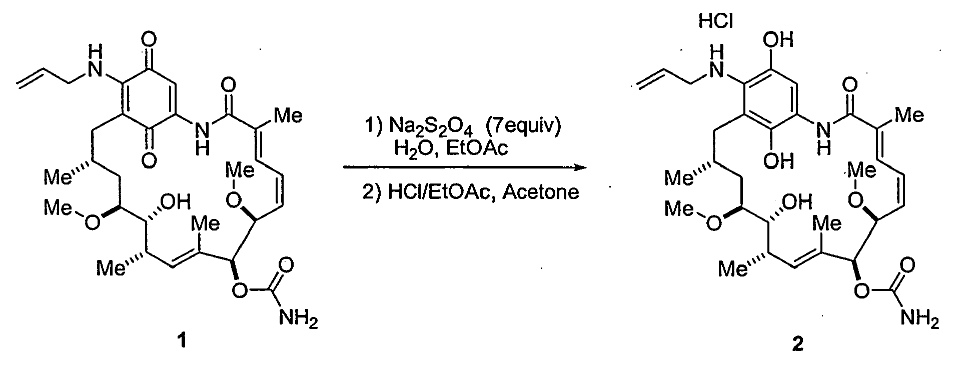

Anhydrous ethyl acetate (170 mL) was added to a flask followed by 17-AAG (8.41 g, 1.44 mmol, 1 equiv). The resultant purple mixture was stirred vigorously under nitrogen. A freshly prepared solution of 10% Na₂S₂O₄ (aq) (1.682 g in 170 mL of deionized water, 10.1 mmol, 7 equiv) was added and the mixture stirred vigorously for 70 min. The color changed from purple to orange indicating a complete reaction. The layers were allowed to separate and the bottom aqueous layer was removed using a separatory funnel. The organic layer was dried with MgSO₄. The drying agent was removed by filtration. The filtrate was transferred to a rotary evaporator flask. Ethyl acetate (50 mL) was used, in portions, to wash the MgSO₄ pad and the wash filtrate was also added to the rotary evaporator flask.

The orange-brown mixture was concentrated on the rotary evaporator to an oil. The remaining ethyl acetate was removed under vacuum.

While this mixture was concentrated, a 5.3 M solution of HCl in ethyl acetate was prepared. Ethyl acetate (16.8 mL) was added to an Erlenmeyer flask and HCl gas bubbled into the stirring mixture for 1 h (with cooling, acetone/wet ice) to achieve saturation. The solution was then warmed to room temperature under a head space of nitrogen.

The oil was dissolved in acetone (252 mL) and transferred to a reaction flask equipped with an addition funnel, a stirrer, a thermometer, and a nitrogen atmosphere. The combined filtrate and rinse were acidified over 5 min to a final pH of 2.5. The resulting slurry was stirred for 18 min at ambient temperature and the solids were then isolated by filtration and washed twice with acetone (84 mL). The solid was then dried under reduced pressure to yield the product

### Example 6

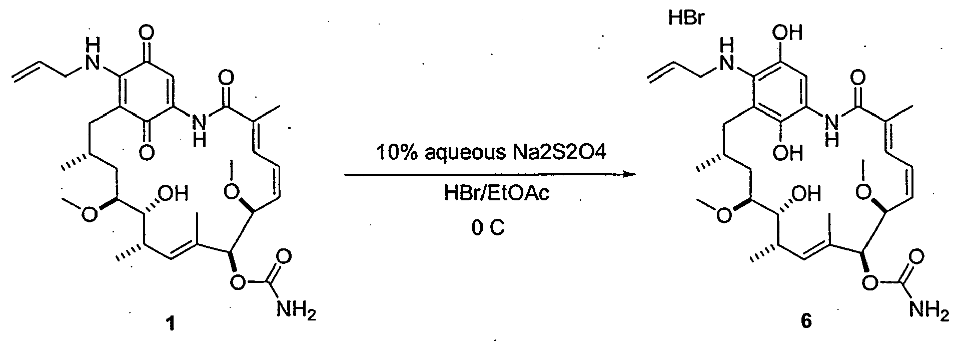

17-Allylamino-17-Demethoxygeldanamycin (1.0 g, 1.71 mmol) in ethyl acetate (20 mL) was stirred vigorously with a freshly prepared solution of 10% aqueous sodium hydrosulfite (2g in 20 mL water) for 30 minutes at ambient temperature. The color changed from dark purple to bright yellow, indicating a complete reaction. The layers were separated and the organic phase was dried with magnesium sulfate (1 g). The reaction solvent was collected and the drying agent was rinsed with ethyl acetate (1 mL). The combined filtrate was cooled to 0 C and acidified with 1.5 M hydrogen bromide in ethyl acetate until a precipitate formed. The resulting slurry was stirred for 30 minutes at ambient temperature. The solids were isolated by filtration, rinsed with ethyl acetate (1 mL) and dried at 40°C, 1 mm Hg, for 16 h to afford 0.352 g (31%) of off white solid.

### Example 7

### Materials and Methods for In Vitro Analysis

### Cell Cultures

The human cancer cell lines SKBr3, MV4-11, K562, SK-MEL-28, LnCAP, and MDA-MB-468 were obtained from the American Type Culture Collection (Manassas, VA). The multiple myeloma RPMI-8226 and MM1.s cells were from Dr. Teru Hideshima (Jerome Lipper Multiple Myeloma Center, Dana Farber Cancer Institute, Boston, MA, USA.). All the cell lines were determined to be mycoplasma-free. The cells were maintained in RPMI-1640 medium supplemented with 10% heat-inactivated FBS, 50 units/mL streptomycin and 50 units/mL penicillin, and incubated at 37°C in 5% CO₂. Adherent cells were dissociated with 0.05% trypsin and 0.02% EDTA in phosphate buffer saline (PBS) without calcium and magnesium prior to plating for experimentation.

### In Vitro Analysis

### MM1.s Cell Cytotoxicity

Alamar Blue assay. MM1.s cells (50,000/well) were incubated for 72 h with increasing concentrations of the test compound. Alamar blue was added to the wells and fluorescence measured 4 h after incubation at 37°C.

### SKBr3 Cell Cytotoxicity

SKBr3 Cells were incubated for 72 h with increasing concentrations of the test compound. For the viability studies Alamar blue was added and wells read after a 6 h incubation.

### MDA-MB-468 Cell Cytotoxicity

MDA-MB-468 Cells were incubated for 72 h with increasing concentrations of the test compound. For the viability studies Alamar blue was added and wells read after 6 h of incubation.

### MV4-11 Cell Cytotoxicity

MV4-11 cells were incubated for 3 days with increasing concentrations of the test compound. Cell viability was assessed using an Alamar blue read out.

### K562 Cell Cytotoxicity

K562 cells were incubated with increasing concentrations of the test compound. Cell viability was assessed using an Alamar blue read out

### SK-MEL-28 Cell Cytotoxicity

Increasing concentrations of the test compound was added to SK-MEL-28 cells in culture for 2, 3 or 4 days and the viability of the cells was measured using Alamar blue.

### LnCAP Cell Cytotoxicity

Increasing concentrations of the test compound added to LnCAP cells in culture for 4 days and the viability of the cells was measured using Alamar blue.

### Example 8

### In Vivo Analysis

### Multiple Myeloma Model

The effects of the test compound were studied in a human multiple myeloma cell line RPMI-8226 in male SCID/NOD mice. In this study, male mice were implanted subcutaneously with RPMI-8226 cells (1 x 10⁷ cells). When the average tumor size reached 100 mm³, animals were randomly assigned to treatment groups (N=10-15/group) to receive either vehicle (50 mM citrate, 50 mM ascorbate, 2.4 mM EDTA adjusted to pH 3.0) or 100 mg/kg (300 mg/m²) of the test compound three consecutive days per week. The test article or vehicle was administered intravenously (IV) via the tail vein in a volume of 0.2 mL over approximately 20 seconds (sec). The animals were sacrificed after 45 days and tumor volumes compared.

### Breast Carcinoma Model

A study was performed in the MDA-MB-468 breast carcinoma model to assess the ability of the test compound to reduce subcutaneous tumor burden. In this study, female nu/nu athymic mice were implanted subcutaneously with MDA-MB-468 cells (1 x 10⁷ cells). When the average tumor size reached 100 mm³, animals were randomly assigned (N=10-15/group) to one of the following treatment groups; vehicle or the test compound at 100 mg/kg (300 mg/m²) twice weekly every week. The test article or vehicle was administered intravenously (IV) via the tail vein in a volume of 0.2 mL over approximately 20 seconds (sec). The animals were sacrificed after 120 days and tumor volumes compared.

### Ovarian Carcinoma Model

A study was performed in the SKOV-3 ovarian mouse xenograft model to assess the ability of the test compound to reduce subcutaneous tumor burden. In this study, female nu/nu athymic mice were implanted subcutaneously with SKOV-3 cells (1 x 10⁷ cells). When the average tumor size reached 100 mm³, animals were randomly assigned to treatment groups (N=10-15/group) to receive either vehicle, the test compound at 100 mg/kg (300 mg/m²) twice weekly. The test article or vehicle was administered intravenously (IV) via the tail vein in a volume of 0.1 mL over approximately 10 seconds (sec). The animals were sacrificed after 88 days and tumor volumes compared.

### Murine Lewis lung model

A study was performed in the mouse Lewis lung model to assess the ability of the compounds disclosed herein to reduce both subcutaneous tumor burden as well as the incidence of lung metastasis. In this study C57B1/6 mice were implanted subcutaneously with Lewis lung cells (1 x 10⁶ cells). When the average tumor size reached 71 mm³ animals (N=10-15/group) were randomly assigned to the following treatment groups: vehicle and Compound 2 75 mg/m² Monday, Wednesday and Friday (MWF) for 3 cycles. Each cycle consisted of 5 days per week of treatment. The test article or vehicle was administered via the tail vein in a volume of 0.2 mL over approximately 30 sec. The animals were sacrificed after 25 days and tumor volumes were compared.

### Prostate Carcinoma

Two studies were performed in mouse PC-3 prostate xenograft models to assess the ability of the test compound to reduce subcutaneous tumor burden as a single agent or in combination with current standard of care. In both studies male nu/nu athymic mice were implanted subcutaneously with PC-3 cells (1 x 10⁷ cells). When the average tumor size reached 100 mm³ animals were randomly assigned to treatment groups (N=10-15/group). In the first study mice received either vehicle; the test compound 100 mg/kg (300mg/m²) twice weekly. The test article or vehicle was administered via the tail vein in a volume of 0.2 mL over approximately 20 sec. The animals were sacrificed after 64 days and tumor volumes compared.

A second study was performed in this model to assess the test compound in combination with the standard of care, Taxotere. In this study separate groups of 10-15 mice each were randomly assigned to receive vehicle, the test compound 100mg/kg (300mg/m²) twice weekly, Taxotere 5 mg/kg (15 mg/m²) once weekly or combination of the test compound with Taxotere. The animals were sacrificed after 64 days and tumor volumes compared.

### Example 9

### Biological Results

The results from the biological activity analysis of the hydroquinones of the invention are presented below. All values are expressed as the mean ± SEM. Data analysis consisted of a one way analysis of variance and if appropriate followed by Dunnets test to assess differences between vehicle and treatment groups. Differences are considered significant at p < 0.05.

| *In Vitro* Results Cell Line | Compound 2 (EC₅₀) | 17-AAG (EC₅₀) |
|---|---|---|
| MM1.s | 307 nM | 306 nM |
| SKBr3 | 32 nM | 34 nM |
| MDA-MB-468 | 335 nM | 356 nM |
| MV4-11 | 25 nM | 38 nM |
| K562 | 29 nM | 50 nM |
| SK-MEL-28 | 200 nM | ------ |
| LnCAP | 73 nM | ------ |

| *In Vivo* Results Cell Line | % Tumor Growth Compared to Vehicle | |
|---|---|---|
| | Compound 2 | Compound 2 + Taxotere |
| RPMI-8226 | 71% | ------- |
| MDA-MB-468 | 76% | ------- |
| SKOV-3 | 59% | ------- |
| Lewis Lung Cell | 60% | ------- |
| PC-3 | 50% | 84% |

| Binding of Compound 2 and 17-AAG to Hsp90 | |
|---|---|
| Compound | Kᵢ |
| Compound 2 | 28 nM |
| 17-AAG | 67 nM |

## Claims

1. Use of a hydroquinone ansamycin compound represented by formula 1: 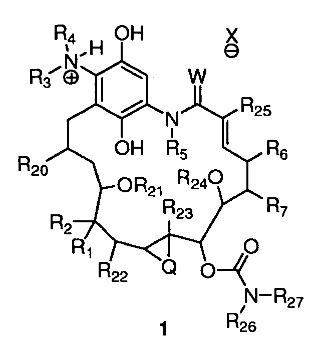 wherein independently for each occurrence:
W is oxygen or sulfur;
Q is oxygen, NR, N(acyl) or a bond;
X⁻ is a conjugate base of a pharmaceutically acceptable acid;
R for each occurrence is independently selected from the group consisting of hydrogen, alkyl, cycloalkyl, heterocycloalkyl, aralkyl, heteroaryl, and heteroaralkyl;
R₁ is hydroxyl, alkoxyl, -OC(O)R₈, -OC(O)OR₉, -OC(O)NR₁₀R₁₁, -OSO₂R₁₂, -OC(O)NHSO₂NR₁₃R₁₄, -NR₁₃R₁₄, or halide; and R₂ is hydrogen, alkyl, or aralkyl; or R₁ and R₂ taken together, along with the carbon to which they are bonded, represent -(C=O)-, -(C=N-OR)-, -(C=N-NHR)-, or -(C=N-R)-;
R₃ and R₄ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ]-R₁₆; or R₃ taken together with R₄ represent a 4-8 membered optionally substituted heterocyclic ring;
R₅ is selected from the group consisting of H, alkyl, aralkyl, and a group having the formula **1a**: 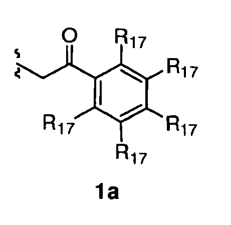
wherein R₁₇ is selected independently from the group consisting of hydrogen, halide, hydroxyl, alkoxyl, aryloxy, acyloxy, amino, alkylamino, arylamino, acylamino, aralkylamino, nitro, acylthio, carboxamide, carboxyl, nitrile, -COR₁₈, - CO₂R₁₈, -N(R₁₈)CO₂R₁₉, -OC(O)N(R₁₈)(R₁₉), -N(R₁₈)SO₂R₁₉, -N(R₁₈)C(O)N(R₁₈)(R₁₉), and -CH₂O-heterocyclyl;
R₆ and R₇ are both hydrogen; or R₆ and R₇ taken together form a bond;
R₈ is hydrogen, alkyl, alkenyl, alkynyl, aryl, cycloalkyl, heterocycloalkyl, aralkyl, heteroaryl, heteroaralkyl, or -[(CR₂)ₚ]-R₁₆;
R₉ is alkyl, alkenyl, alkynyl, aryl, cycloalkyl, heterocycloalkyl, aralkyl, heteroaryl, heteroaralkyl, or -[(CR₂)ₚ]-R₁₆;
R₁₀ and R₁₁ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ]-R₁₆; or R₁₀ and R₁₁ taken together with the nitrogen to which they are bonded represent a 4-8 membered optionally substituted heterocyclic ring;
R₁₂ is alkyl, alkenyl, alkynyl, aryl, cycloalkyl, heterocycloalkyl, aralkyl, heteroaryl, heteroaralkyl, or -[(CR₂)ₚ]-R₁₆;
R₁₃ and R₁₄ are each independently selected from the group consisting of hydrogen, alkyl, alkenyl, alkynyl, aryl, cycloalkyl, heterocycloalkyl, aralkyl, heteroaryl, heteroaralkyl, and -[(CR₂)ₚ]-R₁₆; or R₁₃ and R₁₄ taken together with the nitrogen to which they are bonded represent a 4-8 membered optionally substituted heterocyclic ring;
R₁₆ for each occurrence is independently selected from the group consisting of hydrogen, hydroxyl, acylamino, -N(R₁₈)COR₁₉, -N(R₁₈)C(O)OR₁₉, -N(R₁₈)SO₂(R₁₉), -CON(R₁₈)(R₁₉), -OC(O)N(R₁₈)(R₁₉), -SO₂N(R₁₈)(R₁₉), -N(R₁₈)(R₁₉), -OC(O)OR₁₈, -COOR₁₈, -C(O)N(OH)(R₁₈), -OS(O)₂OR₁₈, -S(O)₂OR₁₈, -OP(O)(OR₁₈)(OR₁₉), -N(R₁₈)P(O)(OR₁₈)(OR₁₉), and -P(O)(OR₁₈)(OR₁₉);
p is 1, 2, 3, 4, 5, or 6;
R₁₈ for each occurrence is independently selected from the group consisting of hydrogen, alkyl, aryl, cycloalkyl, heterocycloalkyl, aralkyl, heteroaryl, and heteroaralkyl;
R₁₉ for each occurrence is independently selected from the group consisting of hydrogen, alkyl, aryl, cycloalkyl, heterocycloalkyl, aralkyl, heteroaryl, and heteroaralkyl; or R₁₈ taken together with R₁₉ represent a 4-8 membered optionally substituted ring;
R₂₀, R₂₁, R₂₂, R₂₄, and R₂₅, for each occurrence are independently alkyl;
R₂₃ is alkyl, -CH₂OH, -CHO, -COOR₁₈, or -CH(OR₁₈)₂;
R₂₆ and R₂₇ for each occurrence are independently selected from the group consisting of hydrogen, alkyl, aryl, cycloalkyl, heterocycloalkyl, aralkyl, heteroaryl, and heteroaralkyl;
and the absolute stereochemistry at a stereogenic center of formula 1 may be R or S or a mixture thereof and the stereochemistry of a double bond may be E or Z or a mixture thereof;
in the manufacture of a medicament for use in the treatment of one or more gastrointestinal stromal tumors in a mammal.

2. Use as claimed in claim 1, wherein said hydroquinone ansamycin compound is represented by the formula 3: 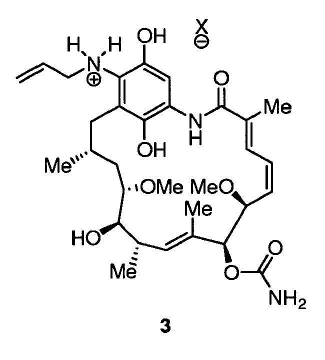 wherein X- is selected from the group consisting of chloride, bromide, iodide, H₂PO4⁻, HSO₄⁻, methylsulfonate, benzenesulfonate, ρ-toluenesulfonate, trifluoromethylsulfonate, 10-camphorsulfonate, naphthalene-1-sulfonic acid-5-sulfonate, ethan-1-sulfonic acid-2-sulfonate, cyclamic acid salt, thiocyanic acid salt, naphthalene-2-sulfonate, and oxalate.

3. Use as claimed in claim 1, wherein said hydroquinone ansamycin compound is a pharmaceutically acceptable salt of 17-allylamino-17-demethoxy-18,21-dihydrogeldanamycin.

4. Use as claimed in claim 4, wherein said hydroquinone ansamycin is the hydrochloride salt of 17-allylamino-17-demethoxy-18,21-dihydrogeldanamycin.

5. Use as claimed in any one of claims 1 to 4, wherein said mammal is a human.

6. Use as claimed in any one of claims 1 to 5, wherein the mode of administration of said hydroquinone ansamycin compound is inhalation, oral, intravenous, sublingual, ocular, transdermal, rectal, vaginal, topical, intramuscular, intraarterial, intrathecal, subcutaneous, buccal, or nasal.

## Patentansprüche

1. Verwendung einer Hydrochinon-Ansamycin-Verbindung, welche durch Formel 1. repräsentiert wird: 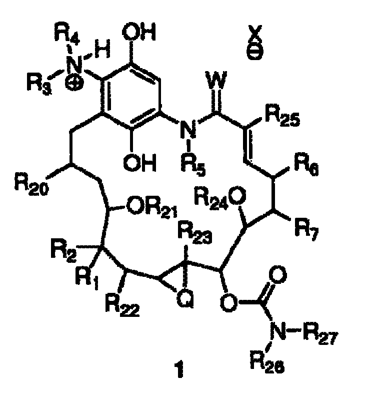 worin für jedes Auftreten unabhängig voneinander:
W Sauerstoff oder Schwefel ist;
Q Sauerstoff, NR, N(Acyl) oder eine Bindung ist;
X" eine konjugierte Base oder eine pharmazeutisch verträgliche Säure ist;
R für jedes Auftreten, unabhängig ausgewählt ist aus der Gruppe, die aus Wasserstoff, Alkyl, Cycloalkyl, Heterocycloalkyl, Aralkyl, Heteroaryl und Heteroaralkyl besteht;
R₁ Hydroxyl, Alkoxyl, -OC(O)R₈, -OC(O)OR₉, -OC(O)NR₁₀R₁₁, -OSO₂R₁₂, -OC (O) NHSO₂NR₁₃R₁₄, -NR₁₃R₁₄ oder Halogen ist; und R₂ Wasserstoff, Alkyl oder Aralkyl ist; oder R₁ und R₂ zusammengenommen, gemeinsam mit dem Kohlenstoff, an welchen sie gebunden sind, -(C=O)-, - (C=N-OR) -, - (C=N-NHR) - oder -(C=N-R)-repräsentieren;
R₃ und R₄ jeweils unabhängig voneinander ausgewählt sind aus der Gruppe, die aus Wasserstoff, Alkyl, Alkenyl, Alkinyl, Aryl, Cycloalkyl, Heterocycloalkyl, Aralkyl, Heteroaryl, Heteroaralkyl und -[(CR₂)ₚ]-R₁₆ besteht; oder R₃ zusammengenommen mit R₄ einen 4-8-gliedrigen, optional substituierten heterocyclischen Ring bildet;
R₅ ausgewählt ist aus der Gruppe, die aus H, Alkyl, Aralkyl und einer Gruppe besteht, welche die Formel 1a besitzt.: 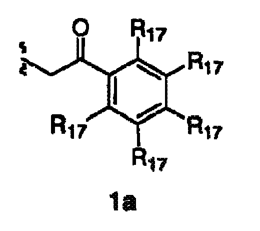
worin R₁₇ unabhängig ausgewählt ist aus der Gruppe, die aus Wasserstoff, Halogen, Hydroxyl, Alkoxyl, Aryloxy, Acyloxy, Amino, Alkylamine, Arylamino, Acylamino, Aralkylamino, Nitro, Acylthio, Carboxamid, Carbonyl, Nitril, -COR₁₈, -CO₂R₁₈, -N-(R₁₈) CO₂R₁₉, -OC(O)N(R₁₈)(R₁₉), -N(R₁₈)SO₂R₁₉, -N(R₁₈)(C)(O)N(R₁₉) und -CH₂O-Heterocyclyl besteht;
R₆ und R₇ beide Wasserstoff sind; oder R₆ und R₇ zusammengenommen eine Bindung bilden;
R₈ Wasserstoff, Alkyl, Alkenyl, Alkenyl, Aryl, Cycloalkyl, Heterocycloalkyl, Aralkyl, Heteroaryl, Heteroaralkyl oder - [ (CR₂) ₚ] -R₁₆ ist;
R₉ Alkyl, Alkenyl, Alkinyl, Acryl, Cycloalkyl, Heterocycloalkyl, Aralkyl, Heteroaryl, Heteroaralkyl, oder - [(CR₂)ₚ]-R₁₆ ist;
R₁₀ und R₁₁ jeweils unabhängig voneinander ausgewählt sind aus der Gruppe, die aus Wasserstoff, Alkyl, Alkenyl, Alkinyl, Aryl, Cycloalkyl, Heterocycloalkyl, Aralkyl, Heteroaryl, Heteroaralkyl und -[(CR₂)ₚ]--R₁₆ besteht; oder R₁₀ und R₁₁ zusammengenommen mit dem Stickstoff, an welchen sie gebunden sind, einen 4-8-gliedrigen, optional substituierten, heterocyclischen Ring bilden;
R₁₂ Alkyl, Alkenyl, Alkenyl, Aryl, Cycloalkyl, Heterocycloalkyl, Aralkyl, Heteroaryl, Heteroaralkyl, oder -[(CR₂)ₚ]-R₁₆ ist;
R₁₃ und R₁₄ jeweils unabhängig voneinander ausgewählt sind aus der Gruppe, bestehend aus Wasserstoff, Alkyl, Alkenyl, Alkenyl, Aryl, Cycloalkyl, Heterocycloalkyl, Aralkyl, Heteroaryl, Heteroaralkyl und -E(CR₂)ₚ]-R₁₆; oder R₁₃ und R₁₄ zusammengenommen mit dem Stickstoff, an welchen sie gebunden sind, einen 4-8-gliedrige, optional substituierten, heterocyclischen Ring repräsentieren;
R₁₆ für jedes Auftreten unabhängig ausgewählt ist aus der Gruppe, die aus Wasserstoff, Hydroxyl, Acylamino, -N(R₁₈)COR₁₉ ; -N(R₁₈)C(O)OR₁₉, -N(R₁₈)SO₂(R₁₉), - CON(R₁₈)(R₁₉), -OC(O)N(R₁₈)(R₁₉), -SO₂N(R₁₈)(R₁₉), -N(R₁₈)(R₁₉), -OC(O)OR₁₈, -COOR₁₈; -C(O)N(OH)(R₁₈), -OS(O)₂OR₁₈, -S(O)₂OR₁₈;
-OP(O)(OR₁₈)(OR₁₉). -N (R₁₈) P (O) (OR₁₈) (OR₁₉) und
- P(O)(OR₁₈)(OR₁₉) besteht;
p 1, 2, 3, 4, 5 oder 6 ist;
R₁₈ für jedes Auftreten unabhängig ausgewählt ist aus der Gruppe, die aus Wasserstoff, Alkyl, Aryl, Cycloalkyl, Heterocycloalkyl, Aralkyl, Heteroaryl und Heteroaralkyl besteht;
R₁₉ für jedes Auftreten unabhängig ausgewählt ist aus der Gruppe, die aus Wasserstoff, Alkyl, Aryl, Cycloalkyl, Heterocycloalkyl, Aralkyl, Heteroaryl und Heteroaralkyl besteht; oder R₁₈ zusammengenommen mit R₁₉ einen 4-8-gliedrigen, optional substituierten Ring bildet;
R₂₀, R₂₁, R₂₂, R₂₄ und R₂₅ unabhängig voneinander Alkyl ist; R₂₃ Alkyl, -CH₂OH, -CHO, -COOR₁₈ oder -CH(OR₁₈)₂ sind;
R₂₆ und R₂₇ für jedes Auftreten unabhängig voneinander ausgewählt sind aus der Gruppe, die aus Wasserstoff, Alkyl, Aryl, Cycloalkyl, Heterocycloalkyl, Aralkyl, Heteroaryl und Heteroaralkyl besteht;
und die absolute Stereochemie an einem stereogenen Zentrum der Formel 1 R oder S oder eine Mischung davon sein kann, und die Stereochemie einer Doppelbindung E oder Z oder eine Mischung davon sein kann;
bei der Herstellung eines Medikaments zur Verwendung bei der Behandlung eines Gastrointestinal-Stromatumors oder mehrerer Gastrointestinal-Stromatumore bei einem Säugetier.

2. Verwendung, wie in Anspruch 1 beansprucht, worin die genannte Hydrochinon-Ansamycin-Verbindung durch die Formel 3 repräsentiert wird: 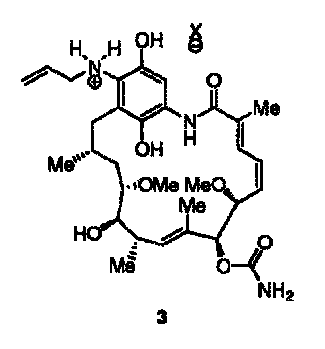 worin X⁻ ausgewählt ist aus der Gruppe, die aus Chlorid, Bromid, Iodid, H₂PO4⁻, HSO₄⁻, Methylsulfonat, Benzolsulfonat, p-Toluolsulfonat, Trifluormethylsulfonat, 10-Camphersulfonat, Naphthalin-1-sulfonsäure-5-sulfonat, Ethan-1-sulfonsäure-2-sulfonat, Cyclaminsäuresalz, Thiocyansäuresalz, Naphthalin-2-sulfonat und Oxalat besteht.

3. Verwendung, wie in Anspruch 1 beansprucht, worin die genannte Hydrochinon-Ansamycin-Verbindung ein pharmazeutisch verträgliches Salz von 17-Allylamino-17-demethoxy-18,21-dihydrogeldanamycin ist.

4. Verwendung wie in Anspruch 4 beansprucht, worin das genannte Hydrochinon-Ansamycin das Hydrochloridsalz von 17-Allylamino-17-demethoxy-18, 21-dihydro-geldanamycin ist.

5. Verwendung, wie in irgendeinem der Ansprüche 1 bis 4 beansprucht, worin das genannte Säugetier ein Mensch ist.

6. Verwendung, wie in irgendeinem der Ansprüche 1 bis 5 beansprucht, worin der Verabreichungsmodus der genannten Hydrochinon-Ansamycin-Verbindung Inhalation, oral, intravenös, sublingual, okular, transdermal, rektal, vaginal, topisch, intramuskulär, intraarteriell, intrathekal, subkutan, bukkal oder nasal ist.

## Revendications

1. Utilisation d'un composé d'ansamycine d'hydroquinone représenté par la formule 1 : 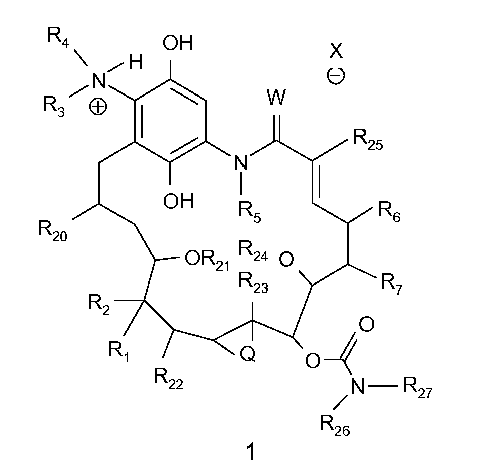 dans laquelle indépendamment pour chaque apparition :
W est l'oxygène ou le soufre ;
Q est l'oxygène, un groupe NR, N(acyle) ou une liaison ;
X- est une base conjuguée d'un sel pharmaceutiquement acceptable ;
R est pour chaque apparition indépendamment choisi dans le groupe constitué de l'hydrogène, d'un groupe alkyle, cycloalkyle, hétérocycloalkyle, aralkyle, hétéroaryle et hétéroaralkyle ;
R₁ est un groupe hydroxyle, alcoxyle, -OC(O)R₈, -OC(O)OR₉, - OC(O)NR₁₀R₁₁, -OSO₂R₁₂, -OC(O)NHSO₂NR₁₃R₁₄, -NR₁₃R₁₄, ou un halogénure ; et R₂ est l'hydrogène, un groupe alkyle ou aralkyle ;
ou R₁ et R₂ pris ensemble avec l'atome de carbone auquel ils sont liés représentent -(C=O)-, -(C=N-OR)-, -(C=N-NHR)-, ou -(C=N-R)- ;
R₃ et R₄ sont chacun indépendamment choisis dans le groupe constitué de l'hydrogène, d'un groupe alkyle, alcényle, alcynyle, aryle, cycloalkyle, hétérocycloalkyle, aralkyle, hétéroaryle, hétéroaralkyle et -[(CR₂)ₚ]-R₁₆ ; ou R₃ pris avec R₄ représentent un noyau hétérocyclique éventuellement substitué à de 4-8 éléments ;
R₅ est choisi dans le groupe constitué de H, d'un groupe alkyle, aralkyle et d'un groupe ayant la formule la : 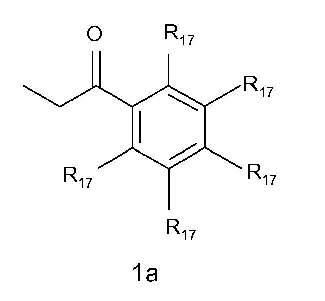
dans laquelle R₁₇ est indépendamment choisi dans le groupe constitué de l'hydrogène, d'un halogénure, d'un groupe hydroxyle, alcoxyle, aryloxy, acyloxy, amino, alkylamino, arylamino, acylamino, aralkylamino, nitro, acylthio, carboxamide, carboxyle, nitrile, -COR₁₈, -CO₂R₁₈, -N(R₁₈)CO₂R₁₉, -OC(O)N(R₁₈)(R₁₉), -N(R₁₈)SO₂R₁₉, -N(R₁₈)C(O)N(R₁₈)(R₁₉), et -CH₂O-hétérocyclyle ;
R₆ et R₇ sont tous deux l'hydrogène ; ou R₆ et R₇ pris ensemble forment une liaison ;
R₈ est l'hydrogène, un groupe alkyle, alcényle, alcynyle, aryle, cycloalkyle, hétérocycloalkyle, aralkyle, hétéroaryle, hétéroaralkyle ou -[(CR₂)ₚ]-R₁₆ ;
R₉ est un groupe alkyle, alcényle, alcynyle, aryle, cycloalkyle, hétérocycloalkyle, aralkyle, hétéroaryle, hétéroaralkyle ou -[(CR₂)ₚ]-R₁₆ ;
R₁₀ et R₁₁ sont chacun indépendamment choisis dans le groupe constitué de l'hydrogène, d'un groupe alkyle, alcényle, alcynyle, aryle, cycloalkyle, hétérocycloalkyle, aralkyle, hétéroaryle, hétéroaralkyle et -[(CR₂)ₚ]-R₁₆ ;
ou R₁₀ et R₁₁ pris ensemble avec l'azote auquel ils sont liés représentent un noyau hétérocyclique éventuellement substitué à 4-8 éléments ;
R₁₂ est un groupe alkyle, alcényle, alcynyle, aryle, cycloalkyle, hétérocycloalkyle, aralkyle, hétéroaryle, hétéroaralkyle, ou -[(CR₂)ₚ]-R₁₆ ;
R₁₃ et R₁₄ sont chacun indépendamment choisis dans le groupe constitué de l'hydrogène, d'un groupe alkyle, alcényle, alcynyle, aryle, cycloalkyle, hétérocycloalkyle, aralkyle, hétéroaryle, hétéroaralkyle et -[(CR₂)ₚ]-R₁₆ ;
ou R₁₃ et R₁₄ pris ensemble avec l'azote auquel ils sont liés représentent un noyau hétérocyclique éventuellement substitué à 4-8 éléments ;
R₁₆ est pour chaque apparition indépendamment choisi dans le groupe constitué de l'hydrogène, d'un groupe hydroxyle, acylamino, -N(R₁₈)COR₁₉, -N(R₁₈)C(O)OR₁₉, -N(R₁₈)SO₂(R₁₉), -CON(R₁₈)(R₁₉), -OC(O)N(R₁₈)(R₁₉), -SO₂N(R₁₈)(R₁₉), -N(R₁₈)(R₁₉), -OC(O)OR₁₈, -COOR₁₈, -C(O)N(OH)(R₁₈), -OS(O)₂OR₁₈, -S(O)₂OR₁₈, -OP(O)(OR₁₈)(OR₁₉), -N(R₁₈)P(O)(OR₁₈)(OR₁₉), et -P(O)(OR₁₈)(OR₁₉) ;
p est égal à 1, 2, 3, 4, 5 ou 6 ;
R₁₈ est pour chaque apparition indépendamment choisi dans le groupe constitué de l'hydrogène, d'un groupe alkyle, aryle, cycloalkyle, hétérocycloalkyle, aralkyle, hétéroaryle et hétéroaralkyle ;
R₁₉ est pour chaque apparition indépendamment choisi dans le groupe constitué de l'hydrogène, d'un groupe alkyle, aryle, cycloalkyle, hétérocycloalkyle, aralkyle, hétéroaryle et hétéroaralkyle ; ou R₁₈ pris avec R₁₉ représentent un noyau éventuellement substitué à 4-8 éléments ;
R₂₀, R₂₁, R₂₂, R₂₄ et R₂₅ sont pour chaque apparition indépendamment un groupe alkyle ;
R₂₃ est un groupe alkyle, -CH₂OH, -CHO, -COOR₁₈ ou -CH(OR₁₈)₂ ;
R₂₆ et R₂₇ sont pour chaque apparition indépendamment choisis dans le groupe constitué de l'hydrogène, d'un groupe alkyle, aryle, cycloalkyle, hétérocycloalkyle, aralkyle, hétéroaryle et hétéroaralkyle ;
et la stéréochimie absolue sur un centre stéréogénique de la formule 1 peut être R ou S ou un mélange de ceux-ci et la stéréochimie d'une double liaison peut être E ou Z ou un mélange de ceux-ci ;
dans la fabrication d'un médicament destiné à être utilisé dans le traitement d'une ou plusieurs tumeurs stromales gastrointestinales chez un mammifère.

2. Utilisation selon la revendication 1, dans laquelle ledit composé d'ansamycine d'hydroquinone est représenté par la formule 3 : 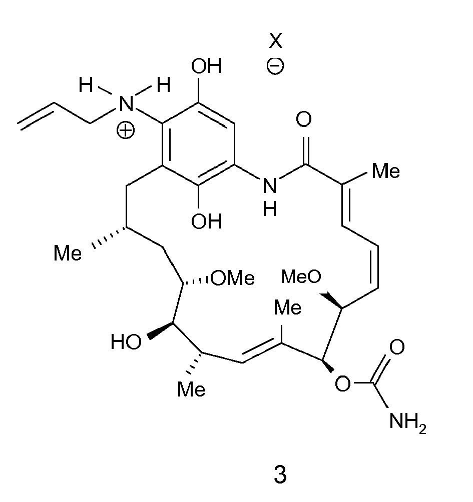 dans laquelle X⁻ est choisi dans le groupe constitué du chlorure, du bromure, de l'iodure, de H₂PO₄⁻ , de HSO₄⁻ , du méthylsulfonate, du benzènesulfonate, du p-toluènesulfonate, du trifluorométhylsulfonate, du 10-camphresulfonate, du naphtalène-1-acide sulfonique-5-sulfonate, de l'éthan-1-acide sulfonique-2-sulfonate, d'un sel d'acide cyclamique, d'un sel d'acide thiocyanique, du naphtalène-2-sulfonate et d'un oxalate.

3. Utilisation selon la revendication 1, dans laquelle ledit composé d'ansamycine d'hydroquinone est un sel pharmaceutiquement acceptable de 17-allylamino-17-diméthoxy-18,21-dihydrogeldanamycine.

4. Utilisation selon la revendication 3, dans laquelle ladite ansamycine d'hydroquinone est le sel d'hydrochlorure de 17-allylamino-17-déméthoxy-18,21-dihydrogeldanamycine.

5. Utilisation selon l'une quelconque des revendications 1 à 4, dans laquelle ledit mammifère est un être humain.

6. Utilisation selon l'une quelconque des revendications 1 à 5, dans laquelle le mode d'administration dudit composé d'ansamycine d'hydroquinone est l'inhalation, est oral, intraveineux, sublingual, oculaire, transdermique, rectal, vaginal, topique, intramusculaire, intra-artériel, intrathécal, sous-cutané, buccal ou nasal.
